(19) Europäisches Patentamt / European Patent Office / Office européen des brevets

(11) **EP 4 393 957 A1**

(12) **EUROPEAN PATENT APPLICATION**
published in accordance with Art. 153(4) EPC

(43) Date of publication:
**03.07.2024 Bulletin 2024/27**

(21) Application number: **22861370.9**

(22) Date of filing: **23.08.2022**

(51) International Patent Classification (IPC):
*C07K 17/02* (2006.01)     *A61K 35/12* (2015.01)
*A61K 45/00* (2006.01)     *A61K 47/69* (2017.01)
*A61P 43/00* (2006.01)     *C07K 7/06* (2006.01)
*C07K 7/08* (2006.01)     *C07K 14/705* (2006.01)
*C12N 5/071* (2010.01)     *C12N 5/0735* (2010.01)
*C12N 5/0775* (2010.01)     *C12N 15/11* (2006.01)
*C12Q 1/24* (2006.01)     *G01N 33/53* (2006.01)

(52) Cooperative Patent Classification (CPC):
A61K 35/12; A61K 45/00; A61K 47/69;
A61P 43/00; C07K 7/06; C07K 7/08; C07K 14/705;
C07K 17/02; C12N 5/06; C12N 15/11; C12Q 1/24;
G01N 33/53

(86) International application number:
**PCT/JP2022/031768**

(87) International publication number:
**WO 2023/027082 (02.03.2023 Gazette 2023/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.08.2021   JP 2021135828**

(71) Applicant: SEKISUI CHEMICAL CO., LTD.
**Osaka-shi
Osaka
530-8565 (JP)**

(72) Inventor: **TAN, Zheli
Tsukuba-shi, Ibaraki 300-4247 (JP)**

(74) Representative: **Ter Meer Steinmeister & Partner
Patentanwälte mbB
Nymphenburger Straße 4
80335 München (DE)**

Remarks:
The complete document including Reference
Table(s) and the Sequence Listing(s) can be
downloaded from the EPO website

(54) **PEPTIDE-BOUND HYBRID LIPOSOME EXOSOME, PEPTIDE-BOUND EXOSOME, COMPOSITION CONTAINING THESE, AND METHOD FOR FORMING SAME**

(57)     To provide a hybrid liposome exosome that can deliver an active component with a higher concentration to target cells, and the like.

According to a peptide-binding hybrid liposome exosome comprising in a lipid bilayer an exosome marker protein selected from CD63, CD81, and CD9 and peptide-binding lipid derivatives in which a peptide binds to a hydrophilic part of a complex lipid, it is possible to impart an additional function regarding endosome release from the peptide-binding hybrid liposome exosome after being incorporated into endosomes of target cells, and thereby it is possible to provide a carrier for a drug delivery system that can deliver an active component with a higher concentration into target cells.

EP 4 393 957 A1

(a)

Beads - Exosome NoFusion No-pep

(A)

Fig. 10 (1 of 12)

**Description**

TECHNICAL FIELD

Priority is claimed on Japanese Patent Application No. 2021-135828, the content of which is incorporated herein by reference.

The present invention relates to a peptide-binding hybrid liposome exosome obtained by fusing an exosome with a liposome, a peptide-binding hybrid liposome exosome-containing composition, and a method of forming a peptide-binding hybrid liposome exosome.

BACKGROUND ART

It is generally known that, when a drug having pharmacological effects is administered to a human body, it inevitably causes undesirable side effects in addition to intended medicinal effects of the drug. In order to reduce side effects of such drugs and increase the efficacy, in recent years, a drug delivery system (DDS) has been proposed. When the drug delivery system is used, since the drug of interest is selectively delivered to the lesion, the efficacy can be exhibited with a lower dosage, and a possibility of the drug being delivered to body parts other than the lesion and undesired side effects being caused can be reduced. Here, regarding application of nanostructures using organic polymers to the drug delivery system, how to impart a controlled release function (sustained release) and a targeting function (cell tropism/affinity) in the blood to capsules encapsulating drugs (called "carriers") is the key for development.

It has been pointed out that liposomes, which are nanoparticles composed of phospholipids, and the like are highly useful as carriers used in such a drug delivery system. Liposomes encapsulating drugs have an advantage that they can be relatively easily formed by mixing drugs and phospholipids in vitro, and have become widely used components in the field of drug delivery systems. However, since liposomes are reconstructed in vitro, it is difficult to impart a targeting function to target cells on liposome themselves.

Incidentally, in recent years, it has become known that a type of vesicle called an exosome is released from cells. Fig. 2 is a diagram illustrating an endosome formation and cell incorporation state. Exosomes are lipid bilayer vesicles with a diameter of 20 nm to 150 nm. As shown in Fig. 2, exosomes encapsulate proteins and nucleic acids such as miRNA and mRNA therein, and have proteins on their surfaces. Exosomes are generally formed in multi-vesicular bodies (A in Fig. 2) formed within cells by budding from the cytoplasm toward the lumen of the multi-vesicular bodies, and are released to the outside of the cells by the fusion of the multi-vesicular bodies and the cell membrane (B in Fig. 2). As shown in C in Fig. 2, exosomes contain labeled molecules derived from the origin cells, and have tropism according to characteristics of the origin cells, and are thought to be involved in physiological effects such as blood coagulation and intercellular signal transmission. When such exosomes come into contact with target cells, they are incorporated into the target cells by endocytosis, the lipid bilayers of endosomes and exosomes are fused, and proteins and nucleic acids such as miRNA and mRNA encapsulated in the exosomes are released into the cytoplasm (D in Fig. 2).

Here, an attempt has been made to apply these exosomes to drug delivery systems, and selectively deliver drugs to specific cells and tissues using targeting of exosomes. However, when proteins and nucleic acid molecules are inserted into exosomes, since the lipid bilayer constituting the walls of the exosomes does not penetrate hydrophilic molecules such as proteins and nucleic acids or molecules with hydrophilic surfaces, there is a problem that active components cannot be effectively encapsulated in the lumen of exosomes. In order to address such a problem, for example, PTL 1 discloses a hybrid liposome exosome in which a liposome encapsulating a physiologically active substance and an exosome are combined, wherein the exosome is a vesicle released from cells, has a diameter of 30 nm to 200 nm, and contains phospholipids, cholesterol, proteins and nucleic acids. According to the invention described in PTL 1, when the liposome and the exosome are combined, a substance to be encapsulated in the liposome can also be encapsulated in the exosome.

PRIOR ART DOCUMENT

Patent Document

[Patent document 1] Japanese Patent No. 6137894

SUMMARY OF INVENTION

However, the invention disclosed in PTL 1 relates to an initial study of a drug delivery system using a hybrid liposome exosome. The hybrid liposome exosome disclosed in PTL 1 is expected to have relatively improved targeting, but has a problem that it is not possible to deliver an active component with a sufficient concentration to target cells for practical use as a carrier for a drug delivery system.

Therefore, the present invention has been made in view of the above circumstances, and an object of the present invention is to provide a hybrid liposome exosome that can deliver an active component with a higher concentration to target cells, and the like.

The inventors of the present invention conducted extensive studies in view of the above circumstances. As a result, they found that the above problems can be addressed using a peptide-binding hybrid liposome exosome comprising peptide-binding lipid derivatives in which a peptide binds to a hydrophilic part of a complex lipid in a lipid bilayer, and completed the present invention. Specifically, the present invention provides the following aspects.

A first aspect of the present invention is a peptide-binding hybrid liposome exosome comprising in a lipid bilayer an exosome marker protein selected from CD63, CD81, and CD9 and peptide-binding lipid derivatives in which a peptide binds to a hydrophilic part of a complex lipid.

According to the first aspect of the present invention, the peptide-binding hybrid liposome exosome comprises in a lipid bilayer peptide-binding lipid derivatives in which peptide binds to a hydrophilic part of a complex lipid. Since the peptide-binding lipid derivatives are easily exposed on the outer surface of the peptide-binding hybrid liposome exosome, it is possible to impart an additional function related to incorporation into target cells to the peptide-binding hybrid liposome exosome, and thereby it is possible to provide a carrier for a drug delivery system that can deliver an active component with a higher concentration into target cells.

A second aspect of the present invention is the peptide-binding hybrid liposome exosome according to the first aspect, wherein the peptide has 10 to 30 amino acid residues, the peptide comprises a repeating sequence composed of 3 to 4 amino acid residues comprising at least one hydrophobic amino acid residue selected from an alanine residue, a leucine residue, and a methionine residue and at least one amino acid residue selected from a glutamic acid residue, an aspartic acid residue, a glutamine residue, and an asparagine residue, and wherein at least one residue of amino acid residues constituting the repeating sequence is one of a glutamic acid residue, an aspartic acid residue, a glutamine residue, and an asparagine residue.

According to the second aspect of the present invention, the peptide comprises a repeating sequence composed of 3 to 4 amino acid residues comprising a predetermined hydrophobic amino acid residue, an acidic amino acid residue and/or a polar amino acid residue, and at least one residue of amino acid residues constituting the repeating sequence is one of a glutamic acid residue, an aspartic acid residue, a glutamine residue, and an asparagine residue. Therefore, the peptide tends to form an $\alpha$-helix structure with a hydrophobic surface under acidic conditions, and due to the action of the $\alpha$-helix structure with a hydrophobic surface, the peptide-binding hybrid liposome exosome after endosome incorporation is more susceptible to endosome release, and incorporation of the contents of the peptide-binding hybrid liposome exosome into the cytoplasm can be promoted.

A third aspect of the present invention is the peptide-binding hybrid liposome exosome according to the first aspect, wherein the peptide has 10 to 30 amino acid residues, and the peptide has at least one basic amino acid residue.

According to the third aspect of the present invention, since the peptides constituting peptide-binding lipid derivatives have 10 to 30 amino acid residues and at least one basic amino acid residue, when the basic group of the basic amino acid residue interacts with the phosphate group on the surface layer of the lipid bilayer under acidic conditions, the peptide-binding hybrid liposome exosome after endosome incorporation is more susceptible to endosome release, and incorporation of the contents of the peptide-binding hybrid liposome exosome into cells can be promoted.

A fourth aspect of the present invention is the peptide-binding hybrid liposome exosome according to the third aspect, wherein the peptide has 2 to 5 consecutive basic amino acid residues.

According to the fourth aspect of the present invention, in the third aspect, since the basic amino acid residues in the peptide are 2 to 5 consecutive residues, when the basic groups of these consecutive basic amino acid residues interact more strongly with the phosphate groups on the surface layer of the lipid bilayer under acidic conditions, incorporation of the peptide-binding hybrid liposome exosome into cells can be further promoted.

A fifth aspect of the present invention is the peptide-binding hybrid liposome exosome according to the third or fourth aspect, wherein the peptide does not contain an acidic amino acid residue.

According to the fifth aspect of the present invention, in the third or fourth aspect, since the peptide does not contain an acidic amino acid residue, the interaction between the basic amino acid residue in the peptide and the phosphate group on the surface layer of the lipid bilayer is not inhibited, and incorporation of the peptide-binding hybrid liposome exosome into cells can be further promoted.

A sixth aspect of the present invention is the peptide-binding hybrid liposome exosome according to any one of the first to fifth aspects, wherein the peptide is any of peptides having amino acid sequences shown in SEQ ID NOs: 1 to 5.

According to the sixth aspect of the present invention, since the peptide used in any one of the first to fifth aspects has amino acid sequences shown in SEQ ID NOs: 1 to 5, incorporation of the peptide-binding hybrid liposome exosome into cells can be further promoted.

A seventh aspect of the present invention is the peptide-binding hybrid liposome exosome according to any one of the first to sixth aspects, wherein the complex lipid is a complex lipid having, at an end, any reactive group selected from the group consisting of primary amino groups, carboxyl groups, hydroxy groups, phosphate groups, and thiol groups, and the complex lipid is at least one selected from the group consisting of sphingophospholipids, glycosphingolipids, glycerophospholipids, glyceroglycolipids, ether type phospholipids, and ether type glycolipids.

The sphingophospholipid, glycosphingolipid, glycerophospholipid, glyceroglycolipid, ether type phospholipid, and ether type glycolipid have polar groups derived from phosphate groups and polar groups derived from sugars in their molecules. According to the seventh aspect of the present invention, since such phosphate groups or polar groups derived from polar groups can react with polar groups of the peptide, the complex lipid and the peptide can be easily bonded.

An eighth aspect of the present invention is the peptide-binding hybrid liposome exosome according to any one of the first to seventh aspects, wherein the peptide and the complex lipid are bonded by a carbodiimide crosslinking reaction, an NHS ester crosslinking reaction, or an imidoester crosslinking reaction.

In the eighth aspect of the present invention, the peptide and the complex lipid are bonded by a carbodiimide crosslinking reaction, an NHS ester crosslinking reaction, or an imidoester crosslinking reaction. According to these chemical reaction methods, the amino group of the peptide and the polar group contained in the complex lipid such as an amino group or a carboxyl group can be stably bonded.

A ninth aspect of the present invention is the peptide-binding hybrid liposome exosome according to any one of the first to eighth aspects, wherein the exosome is an exosome obtained from stem cells or an exosome obtained from at least one selected from the group consisting of pluripotent stem cells, mesenchymal stem cells, ectoderm-derived cells, mesoderm-derived cells, and endoderm-derived cells.

In the ninth aspect of the present invention, the exosome is selected from the group consisting of pluripotent stem cells, mesenchymal stem cells, ectoderm-derived cells, mesoderm-derived cells, and endoderm-derived cells. That is, since the peptide-binding hybrid liposome exosome according to any one of the first to eighth aspects of the present invention can be directly applied to exosomes derived from cells with various properties, targeting to various cells can be imparted to the peptide-binding hybrid liposome exosome depending on properties of the exosome.

A tenth aspect of the present invention is a method of forming a peptide-binding hybrid liposome exosome, the method comprising a step of binding a peptide to a hydrophilic part of a complex lipid to form peptide-binding lipid derivatives, a step of mixing a phospholipid composition containing a phospholipid and the peptide-binding lipid derivatives to form a liposome into which the peptide-binding lipid derivatives are incorporated, and a step of fusing an exosome with the liposome to form a peptide-binding hybrid liposome exosome.

In the method of forming a peptide-binding hybrid liposome exosome according to the tenth aspect of the present invention, peptide-binding lipid derivatives are formed, these are mixed with a phospholipid composition containing a phospholipid to form a liposome into which the peptide-binding lipid derivatives are incorporated, and an exosome is fused with the liposome to form a peptide-binding hybrid liposome exosome. Therefore, the peptide-binding lipid derivatives can be easily introduced into the peptide-binding hybrid liposome exosome without applying a strong load, and an active component can be easily encapsulated when the liposome is formed.

An eleventh aspect of the present invention is a peptide-binding hybrid liposome exosome formed by the method of forming a peptide-binding hybrid liposome exosome according to the tenth aspect.

According to the eleventh aspect of the present invention, a peptide-binding hybrid liposome exosome similar to the peptide-binding hybrid liposome exosome according to the first aspect of the present invention is obtained. Therefore, it is possible to impart an additional function related to incorporation into target cells to the peptide-binding hybrid liposome exosome, and thereby it is possible to provide a carrier for a drug delivery system that can deliver an active component with a higher concentration into target cells.

A twelfth aspect of the present invention is a peptide-binding hybrid liposome exosome-containing composition obtained by adding first primary antibodies that specifically bind to an exosome marker protein selected from CD63, CD81, and CD9, second primary antibodies that specifically bind to any of peptides having amino acid sequences shown in SEQ ID NOs: 1 to 5, first secondary antibodies conjugated with a first fluorescent dye, which specifically bind to the first primary antibodies, and second secondary antibodies conjugated with a second fluorescent dye, which specifically bind to the second primary antibodies, where the ratio of [the product of the amount of the first primary antibodies added and the antibody titer] and [the product of the amount of the second secondary antibodies added and the antibody titer] is 1:1, the amount ratio of the first primary antibodies and the first secondary antibodies added is 2:1, the amount ratio of the second primary antibodies and the second secondary antibodies added is 2:1, and when a peptide-binding hybrid liposome exosome that binds to a magnetic bead that specifically binds to a complex lipid contained in an exosome is analyzed using flow cytometry, the following condition is satisfied:

$$0.6 \leq A/(A+B+C) \leq 1.0 \quad \text{Formula (1)}$$

(where, in Formula (1), A is an area of a peak indicating the peptide-binding hybrid liposome exosome labeled with both the first fluorescent dye and the second fluorescent dye, B is an area of a peak indicating the exosome labeled with only the first fluorescent dye, and C is an area of a peak indicating the liposome labeled with only the second fluorescent dye).

According to the twelfth aspect of the present invention, a composition containing a peptide-binding hybrid liposome exosome similar to the peptide-binding hybrid liposome exosome according to the first aspect of the present invention is obtained. Therefore, it is possible to impart an additional function related to incorporation into target cells to the peptide-binding hybrid liposome exosome, and thereby it is possible to provide a carrier for a drug delivery system that can deliver an active component with a higher concentration into target cells.

A thirteenth aspect of the present invention is a method of selecting a peptide-binding hybrid liposome exosome-containing composition, the method comprising: adding first primary antibodies that specifically bind to an exosome marker protein selected from CD63, CD81, and CD9, second primary antibodies that specifically bind to any of peptides having amino acid sequences shown in SEQ ID NOs: 1 to 5, first secondary antibodies conjugated with a first fluorescent dye, which specifically bind to the first primary antibodies, and second secondary antibodies conjugated with a second fluorescent dye, which specifically bind to the second primary antibodies, where the ratio of [the product of the amount of the first primary antibodies added and the antibody titer] and [the product of the amount of the second secondary antibodies added and the antibody titer] is 1:1, the amount ratio of the first primary antibodies and the first secondary antibodies added is 2:1, and the amount ratio of the second primary antibodies and the second secondary antibodies added is 2:1; and selecting a peptide-binding hybrid liposome exosome-containing composition that satisfies the following condition when a peptide-binding hybrid liposome exosome that binds to a magnetic bead that specifically binds to a complex lipid contained in an exosome is analyzed using flow cytometry:

$$0.6 \leq A/(A+B+C) \leq 1.0 \quad \text{Formula (1)}$$

(where, in Formula (1), A is an area of a peak indicating the peptide-binding hybrid liposome exosome labeled with both the first fluorescent dye and the second fluorescent dye, B is an area of a peak indicating the exosome labeled with only the first fluorescent dye, and C is an area of a peak indicating the liposome labeled with only the second fluorescent dye).

According to the thirteenth aspect of the present invention, it is possible to select a composition containing a peptide-binding hybrid liposome exosome similar to the peptide-binding hybrid liposome exosome according to the first aspect of the present invention. Therefore, it is possible to impart an additional function related to incorporation into target cells to the peptide-binding hybrid liposome exosome, and thereby it is possible to provide a carrier for a drug delivery system that can deliver an active component with a higher concentration into target cells.

A fourteenth aspect of the present invention is a peptide-binding exosome comprising an exosome comprising in a lipid bilayer an exosome marker protein selected from CD63, CD81, and CD9 and peptide-binding lipid derivatives in which a peptide binds to a hydrophilic part of a complex lipid.

According to the fourteenth aspect of the present invention, the peptide-binding exosome comprises in a lipid bilayer peptide-binding lipid derivatives in which peptide binds to a hydrophilic part of a complex lipid. Since the peptide-binding lipid derivatives are easily exposed on the outer surface of the peptide-binding exosome, it is possible to impart an additional function related to incorporation into target cells to the peptide-binding exosome, and thereby it is possible to provide a carrier for a drug delivery system that can deliver an active component with a higher concentration into target cells.

A fifteenth aspect of the present invention is the peptide-binding hybrid liposome exosome according to the fourteenth aspect, wherein the peptide has 10 to 30 amino acid residues, the peptide comprises a repeating sequence composed of 3 to 4 amino acid residues comprising at least one hydrophobic amino acid residue selected from an alanine residue, a leucine residue, and a methionine residue and at least one amino acid residue selected from a glutamic acid residue, an aspartic acid residue, a glutamine residue, and an asparagine residue, and at least one residue of amino acid residues constituting the repeating sequence is one of a glutamic acid residue, an aspartic acid residue, a glutamine residue, and an asparagine residue.

A fifteenth aspect of the present invention is the peptide-binding hybrid liposome exosome according to the fourteenth aspect, wherein the peptide has 10 to 30 amino acid residues, and the peptide is at least one basic amino acid residue.

A seventeenth aspect of the present invention is the peptide-binding hybrid liposome exosome according to the sixteenth aspect, wherein the peptide has 2 to 5 consecutive basic amino acid residues.

An eighteenth aspect of the present invention is the peptide-binding hybrid liposome exosome according to the sixteenth or seventeenth aspect, wherein the peptide does not contain an acidic amino acid residue.

A nineteenth aspect of the present invention is the peptide-binding hybrid liposome exosome according to any one of the fourteenth to eighteenth aspects, wherein the peptide is any of peptides having amino acid sequences shown in SEQ ID NOs: 1 to 5.

According to the fifteenth to nineteenth aspects of the present invention, in the peptide-binding exosome, the same effects as in the second to sixth aspects of the present invention can be obtained.

A twentieth aspect of the present invention is the peptide-binding hybrid liposome exosome according to any one of the fourteenth to nineteenth aspects, wherein the complex lipid is a complex lipid having, at an end, any reactive group selected from the group consisting of primary amino groups, carboxyl groups, hydroxy groups, phosphate groups, and thiol groups, and the complex lipid is at least one selected from the group consisting of sphingophospholipids, glycosphingolipids, glycerophospholipids, glyceroglycolipids, ether type phospholipids, and ether type glycolipids.

According to the twentieth aspect of the present invention, in the peptide-binding exosome, the same effects as in the seventh aspect of the present invention can be obtained.

According to the present invention, the peptide-binding hybrid liposome exosome comprises in a lipid bilayer peptide-binding lipid derivatives in which peptide binds to a hydrophilic part of a complex lipid. Since the peptide-binding lipid derivatives are easily exposed on the outer surface of the peptide-binding hybrid liposome exosome, it is possible to impart an additional function regarding endosome release from the peptide-binding hybrid liposome exosome after being incorporated into endosomes of target cells, and thereby it is possible to provide a carrier for a drug delivery system that can deliver an active component with a higher concentration into target cells.

BRIEF DESCRIPTION OF DRAWINGS

[Fig. 1]
Fig. 1 is a diagram illustrating an outline of a method of forming a peptide-binding hybrid liposome exosome according to an embodiment and a method of using the same.
[Fig. 2]
Fig. 2 is a diagram illustrating an endosome formation and cell incorporation state.
[Fig. 3]
Fig. 3 is a diagram showing a conceptual diagram of exosome flow cytometry using a phosphatidylserine-binding protein Tim4.
[Fig. 4]
Fig. 4 is a diagram showing the results of an examination of a cell growth inhibitory effect of peptide-binding to anticancer drug-encapsulated liposomes in Experimental Example 1.
[Fig. 5]
Fig. 5 is a diagram showing the results of verification of a growth inhibitory effect of HepG2 cells by siRNA-encapsulated liposomes in Experimental Example 2.
[Fig. 6A]
Fig. 6A is a diagram showing the results of an examination of a cell growth inhibitory effect of peptide-binding to siRNA-encapsulated liposomes in Experimental Example 3.
[Fig. 6B]
Fig. 6B is a diagram showing the results of an examination of a cell growth inhibitory effect of peptide-binding to siRNA-encapsulated liposomes in Experimental Example 3.
[Fig. 6C]
Fig. 6C is a diagram showing the results of an examination of a cell growth inhibitory effect of peptide-binding to siRNA-encapsulated liposomes in Experimental Example 3.
[Fig. 6D]
Fig. 6D is a diagram showing the results of an examination of a cell growth inhibitory effect of peptide-binding to siRNA-encapsulated liposomes in Experimental Example 3.
[Fig. 6E]
Fig. 6E is a diagram showing the results of an examination of a cell growth inhibitory effect of peptide-binding to siRNA-encapsulated liposomes in Experimental Example 3.
[Fig. 6F]
Fig. 6F is a diagram showing the results of an examination of a cell growth inhibitory effect of peptide-binding to siRNA-encapsulated liposomes in Experimental Example 3.
[Fig. 7]
Fig. 7 is a diagram showing the results of an examination of the influence of MSC-derived exosomes on cell growth in Experimental Example 4.
[Fig. 8A]
Fig. 8A is a diagram showing the results of an examination of the influence of peptide-binding MSC-derived exosome solutions on cell growth in Experimental Example 5.

[Fig. 8B]
Fig. 8B is a diagram showing the results of an examination of the influence of peptide-binding HEK293 cell-derived exosome solutions on cell growth in Experimental Example 5.
[Fig. 9A]
Fig. 9A is a diagram showing the results of an examination of the influence of peptide-binding MSC-derived exosome solutions on cell growth in Experimental Example 6.
[Fig. 9B]
Fig. 9B is a diagram showing the results of an examination of the influence of peptide-binding MSC-derived exosome solutions on cell growth in Experimental Example 6.
[Fig. 9C]
Fig. 9C is a diagram showing the results of an examination of the influence of peptide-binding MSC-derived exosome solutions on cell growth in Experimental Example 6.
[Fig. 9D]
Fig. 9D is a diagram showing the results of an examination of the influence of peptide-binding MSC-derived exosome solutions on cell growth in Experimental Example 6.
[Fig. 9E]
Fig. 9E is a diagram showing the results of an examination of the influence of peptide-binding MSC-derived exosome solutions on cell growth in Experimental Example 6.
[Fig. 10]

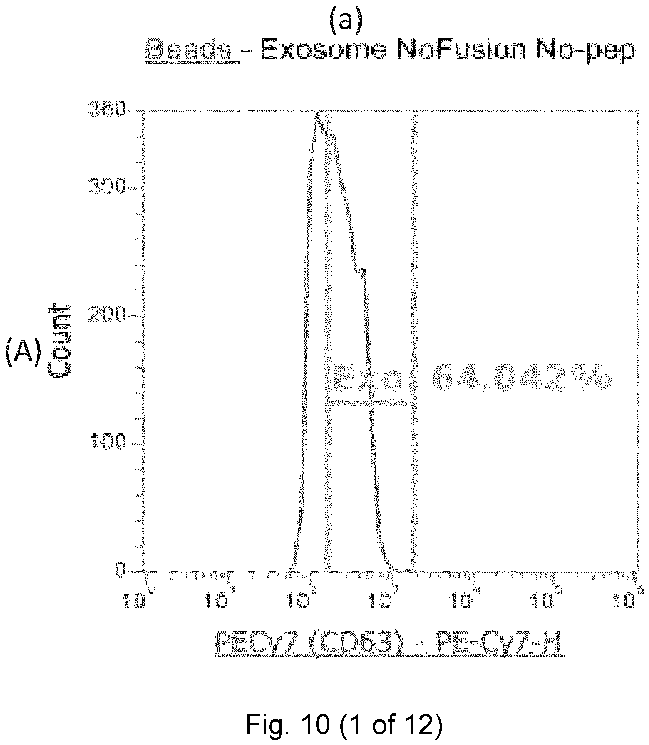

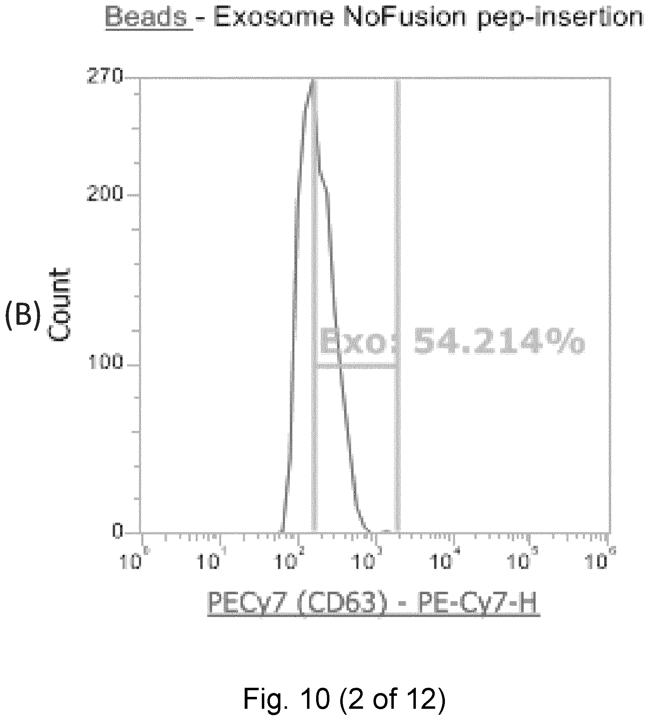

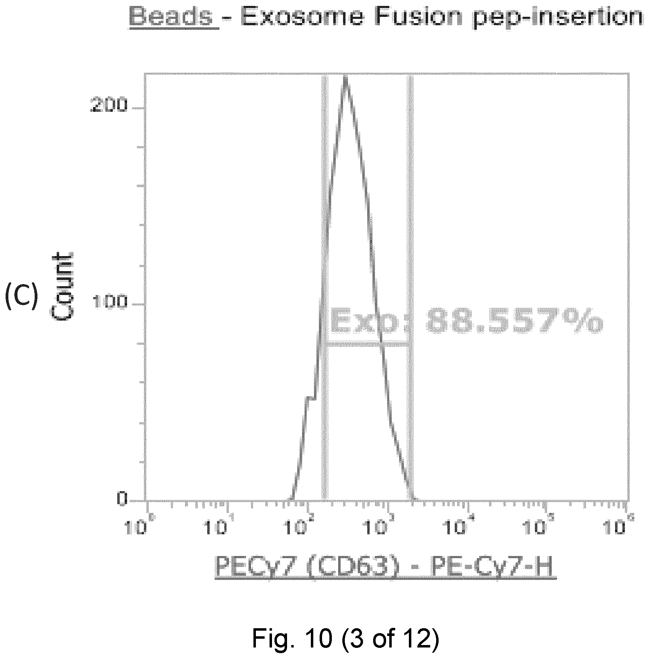

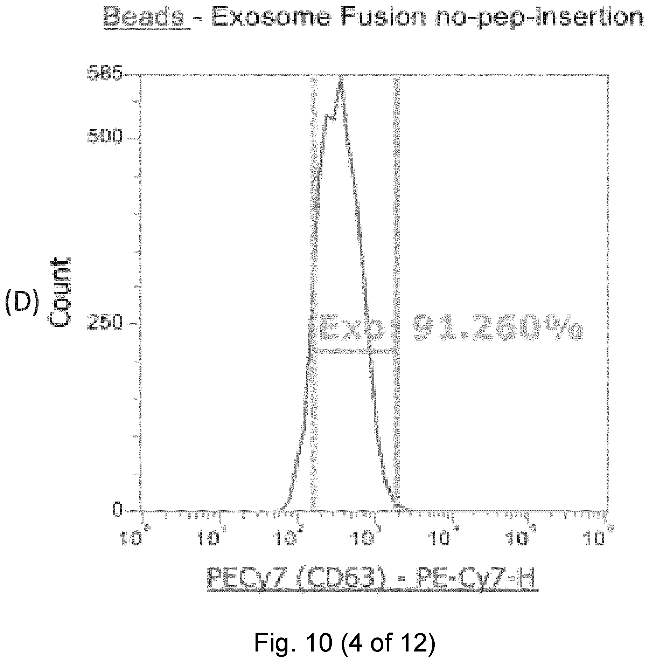

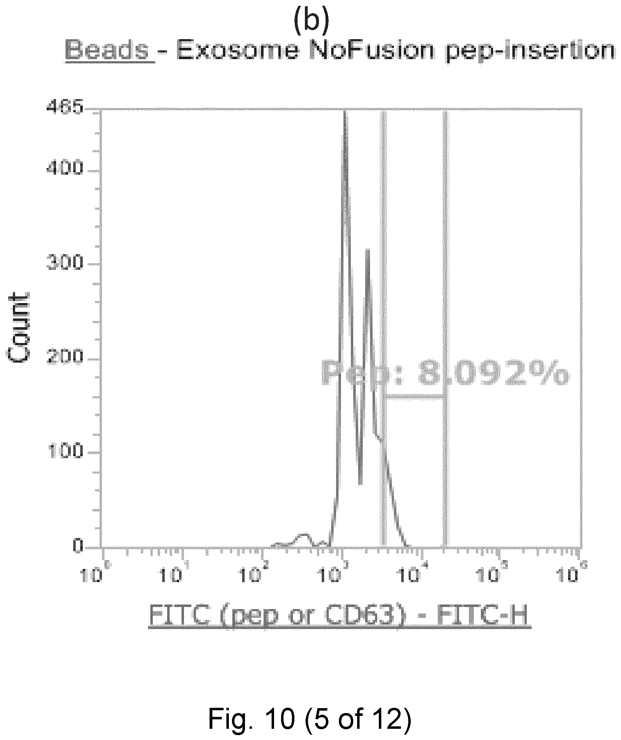

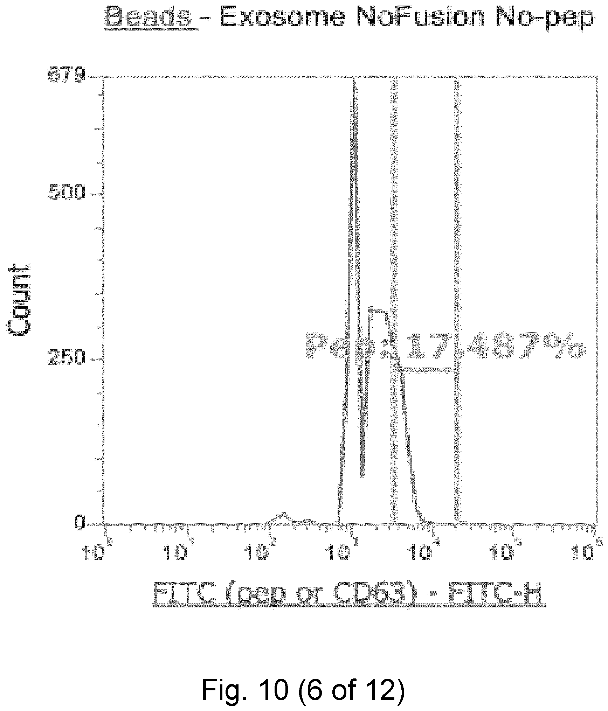

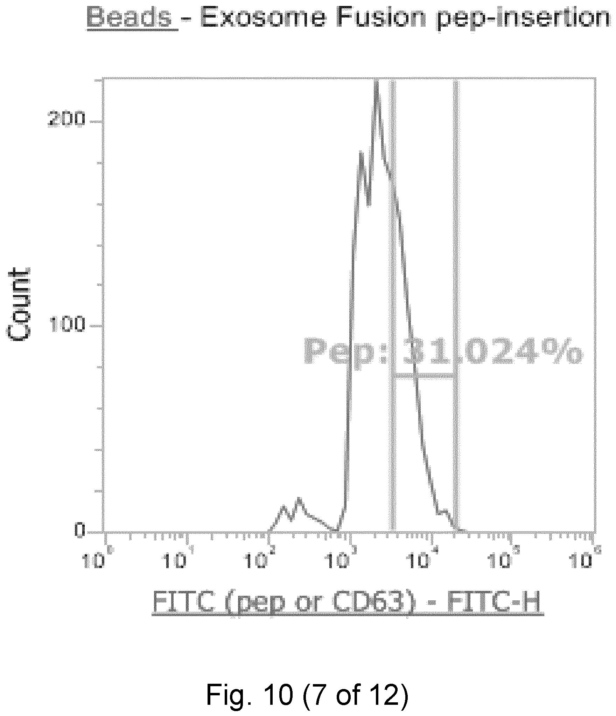

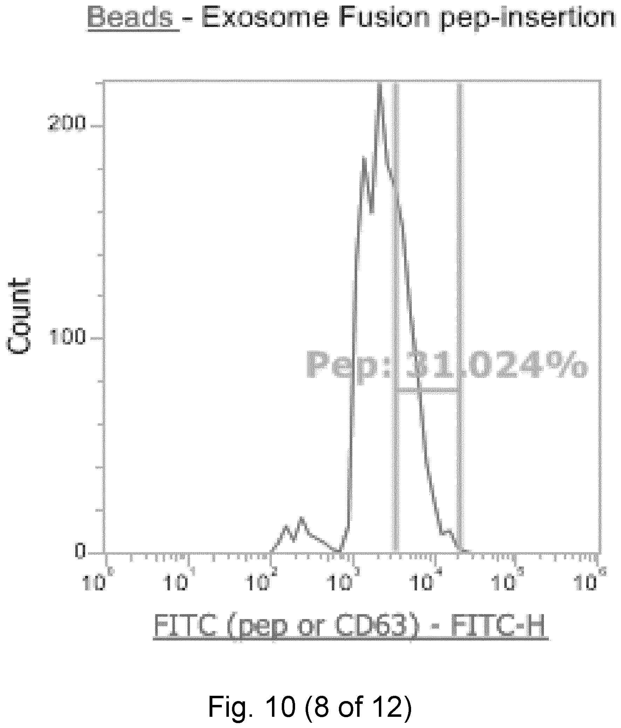

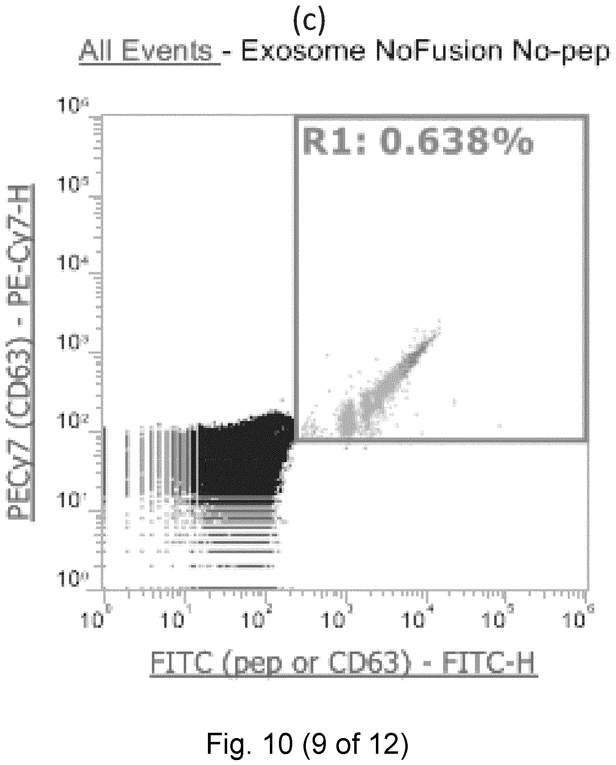

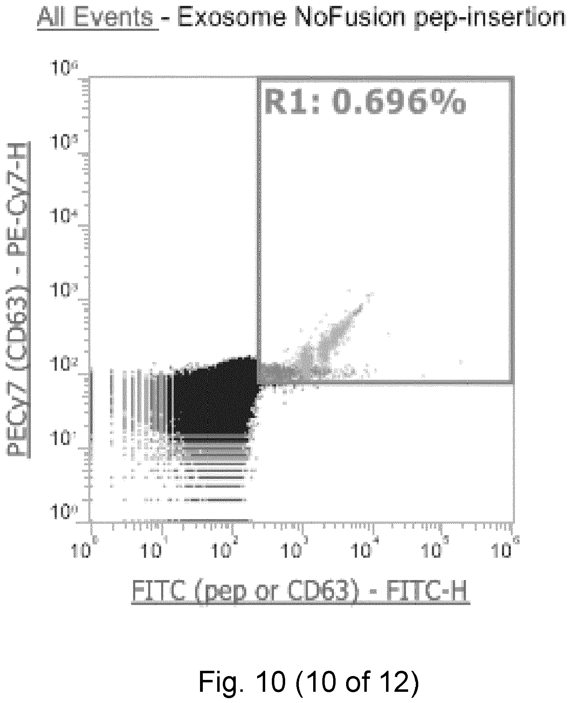

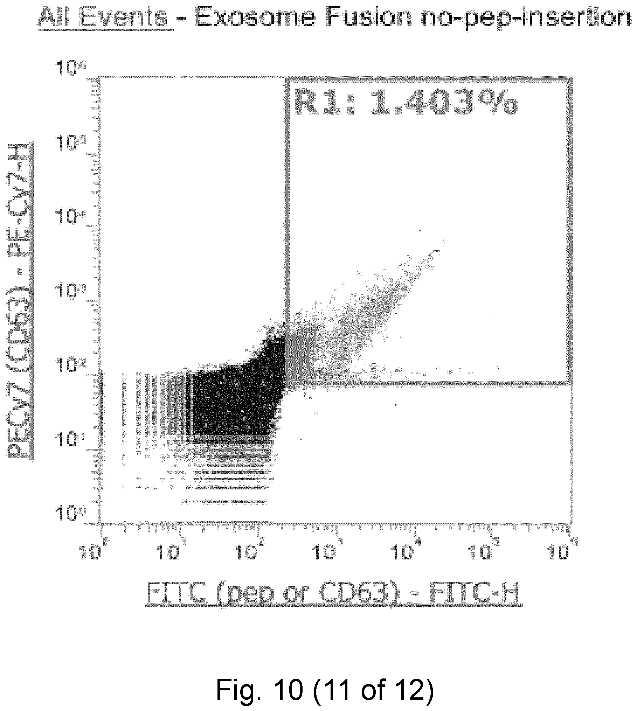

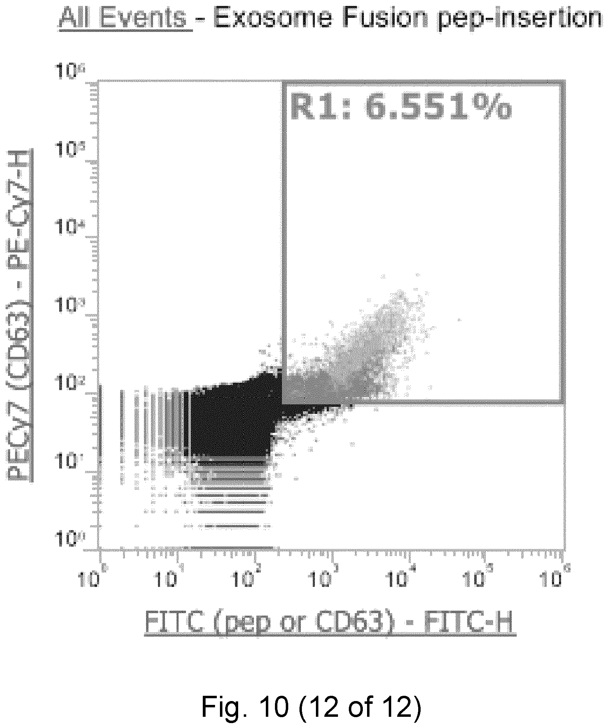

Fig. 10 shows diagrams of the results of flow cytometry of hybrid liposome exosomes of Lipocapsulater FD-U PL to which a peptide shown in SEQ ID NO: 3 was added and an exosome.
[Fig. 11]

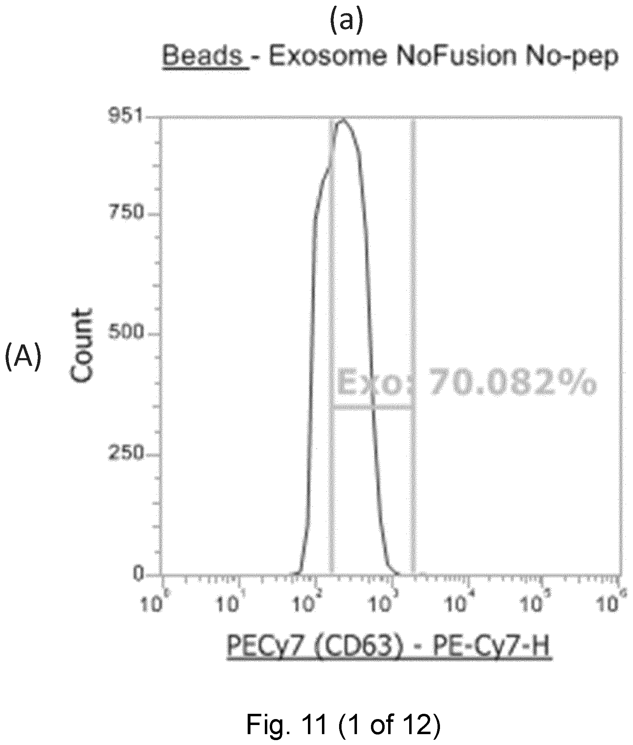

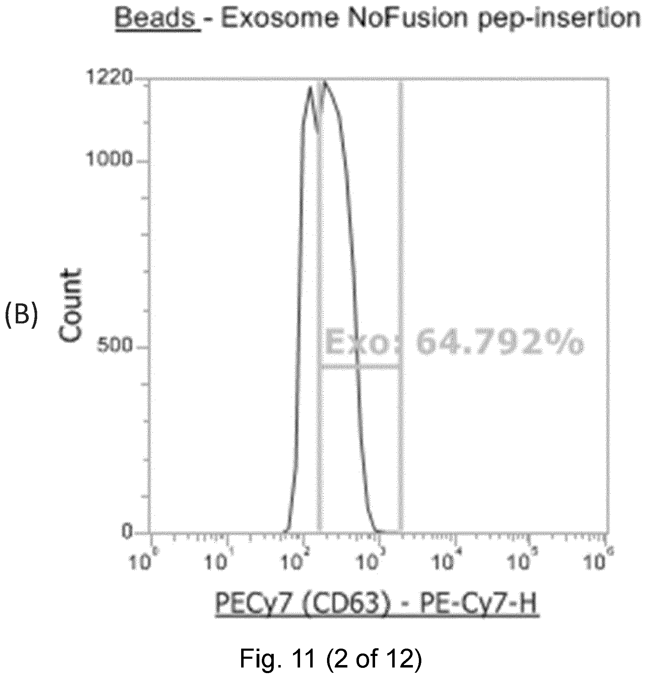

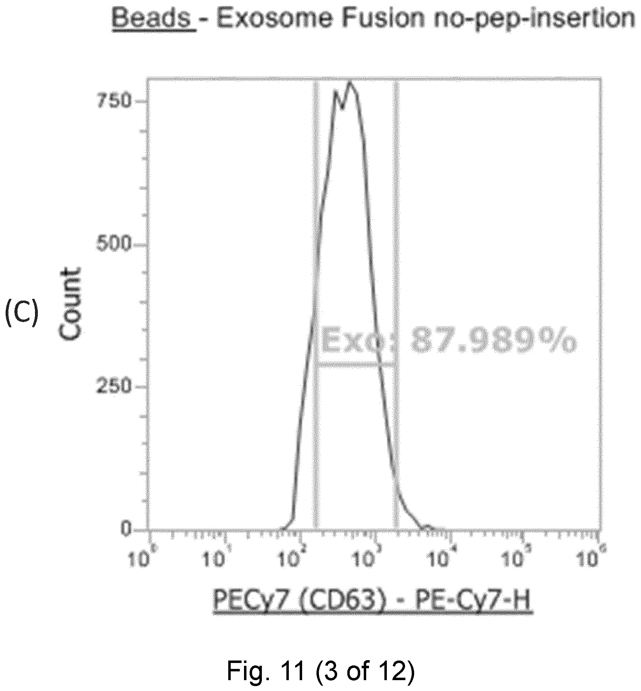

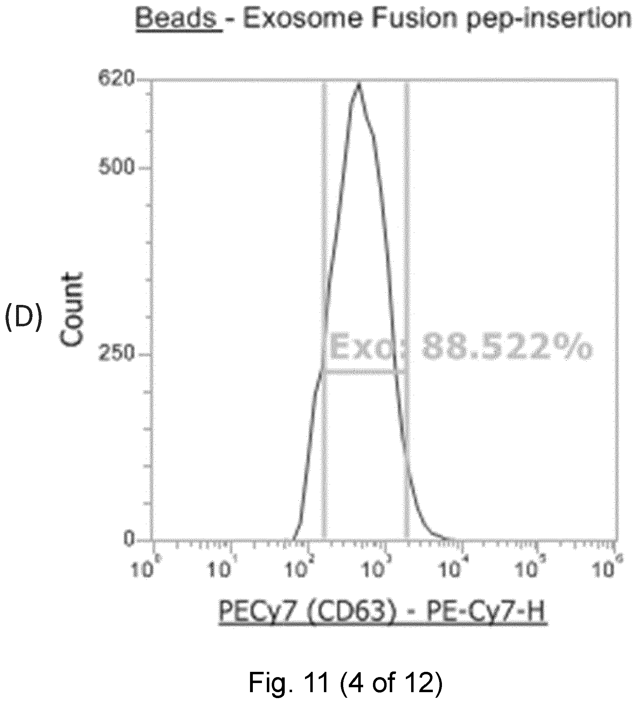

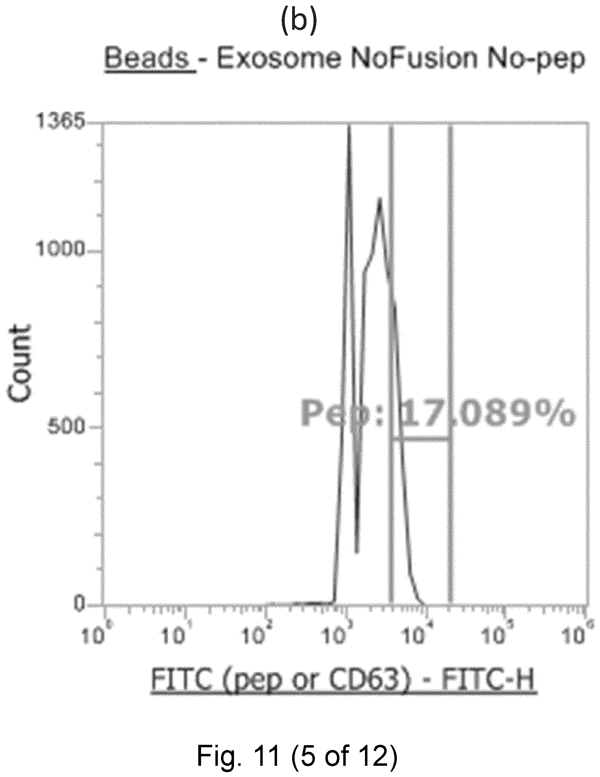

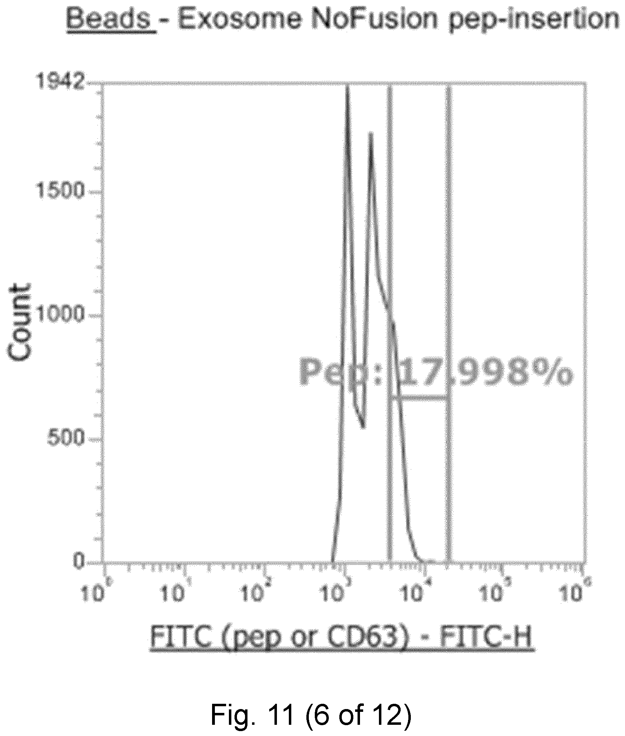

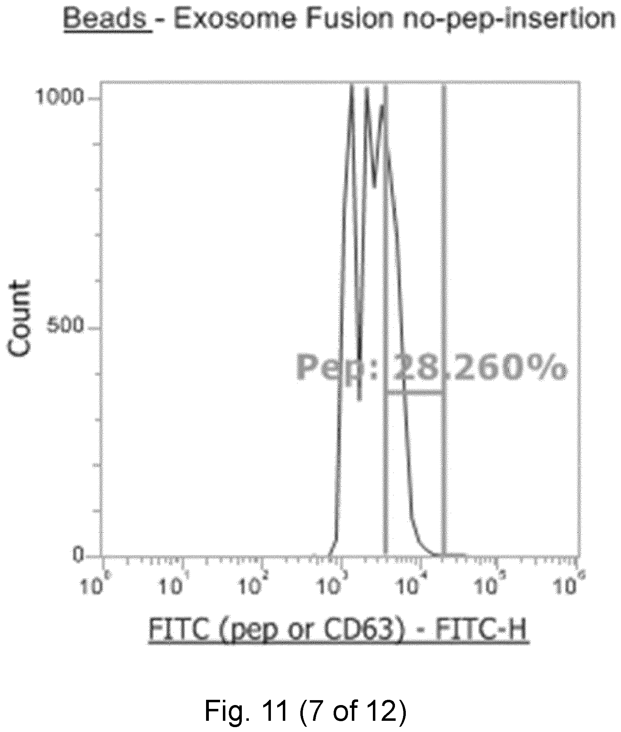

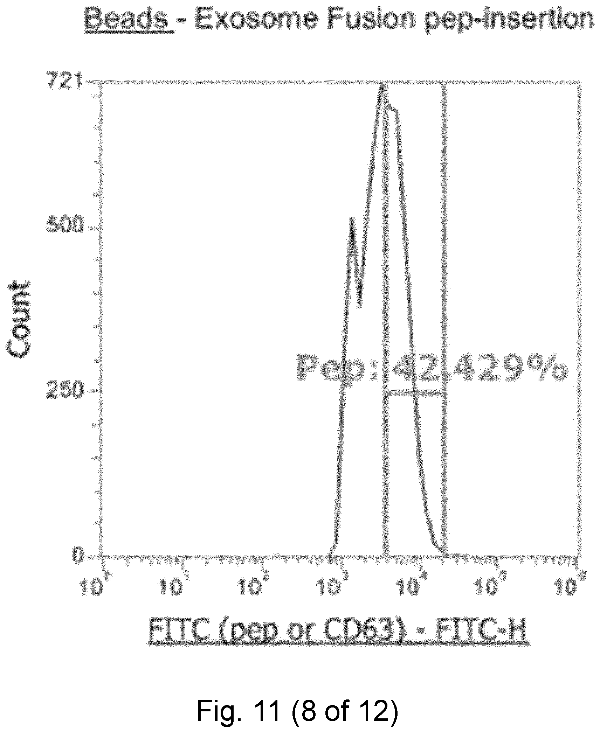

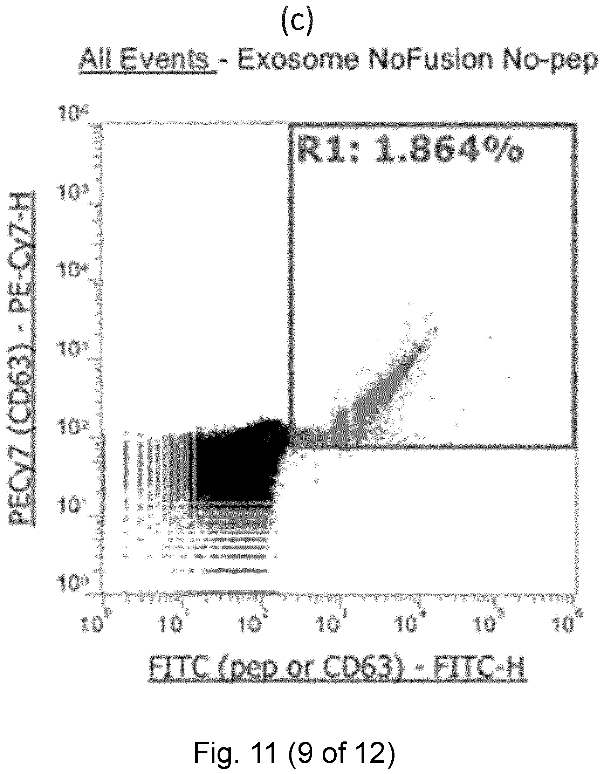

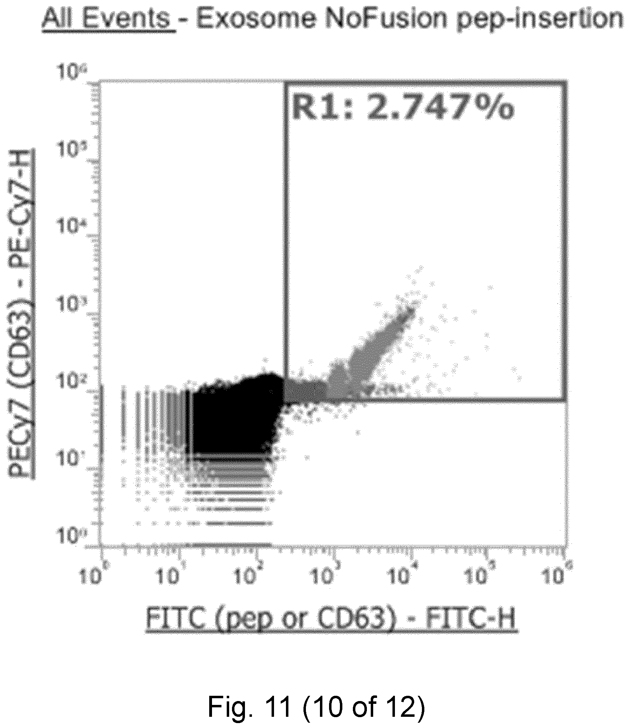

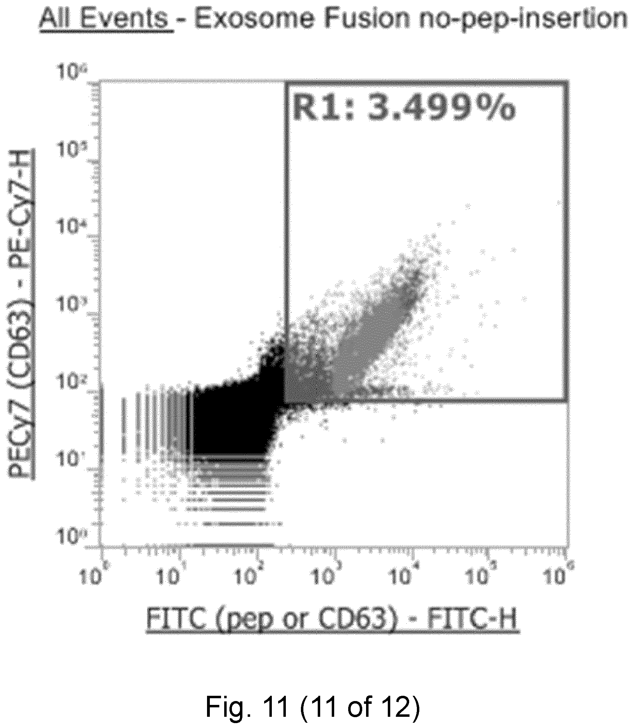

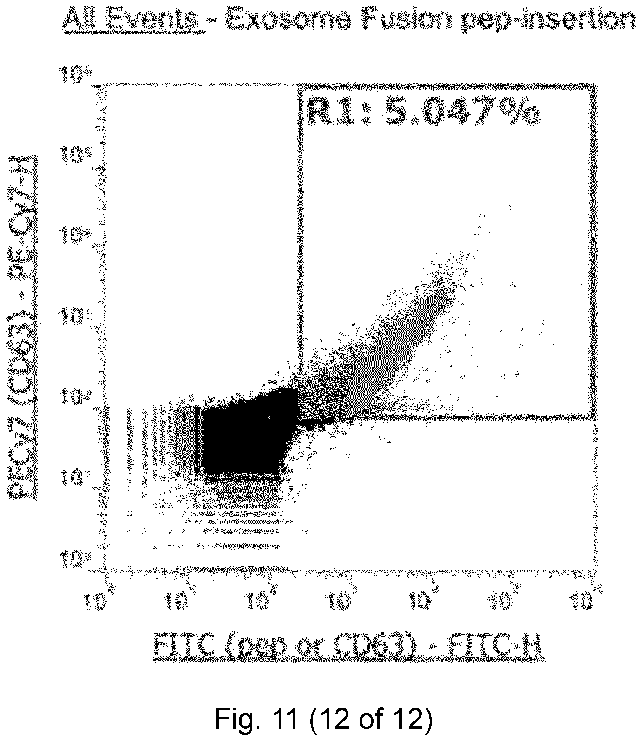

Fig. 11 shows diagrams of the results of flow cytometry of hybrid liposome exosomes of Lipocapsulater FD-U PL to which a peptide shown in SEQ ID NO: 5 was added and an exosome.
[Fig. 12]

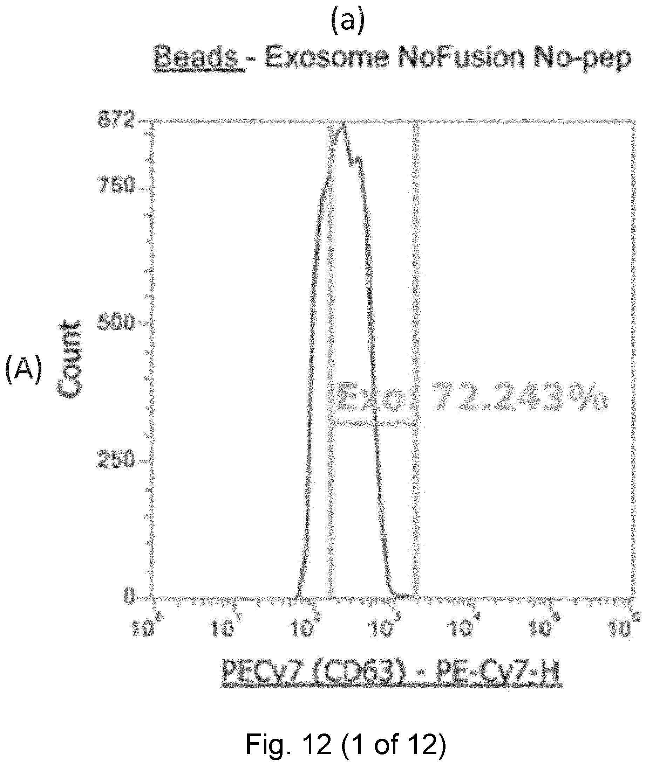

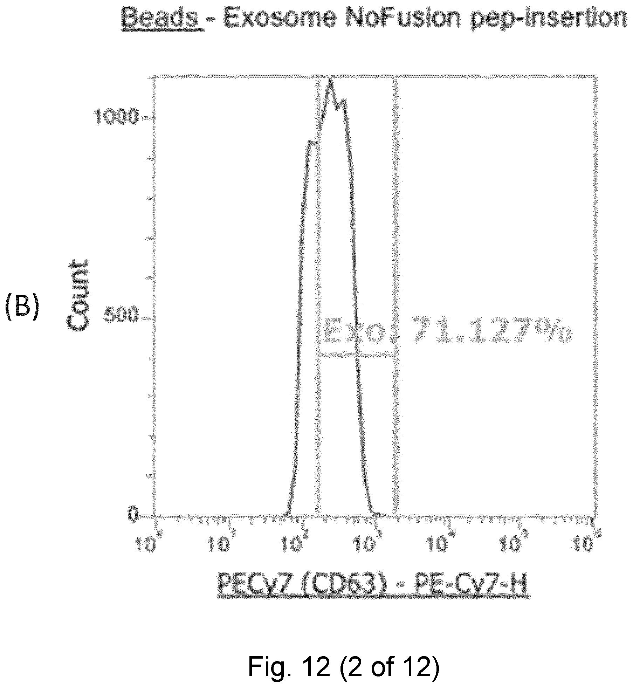

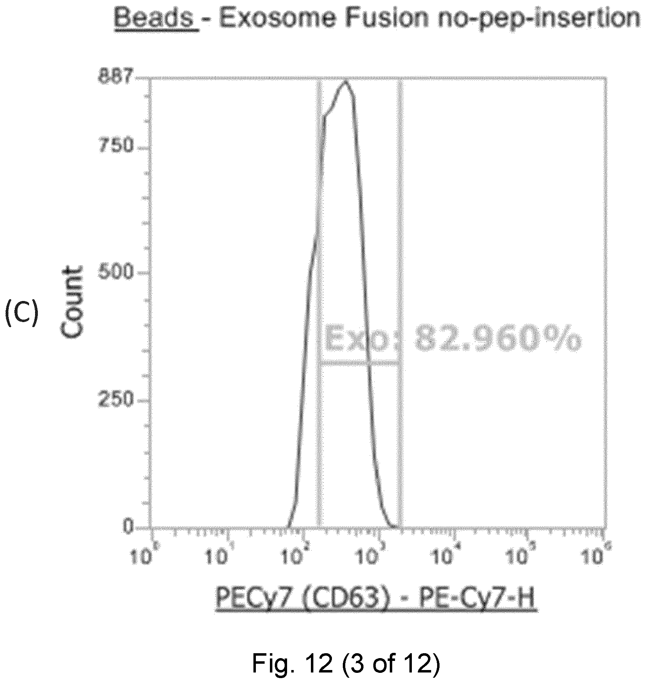

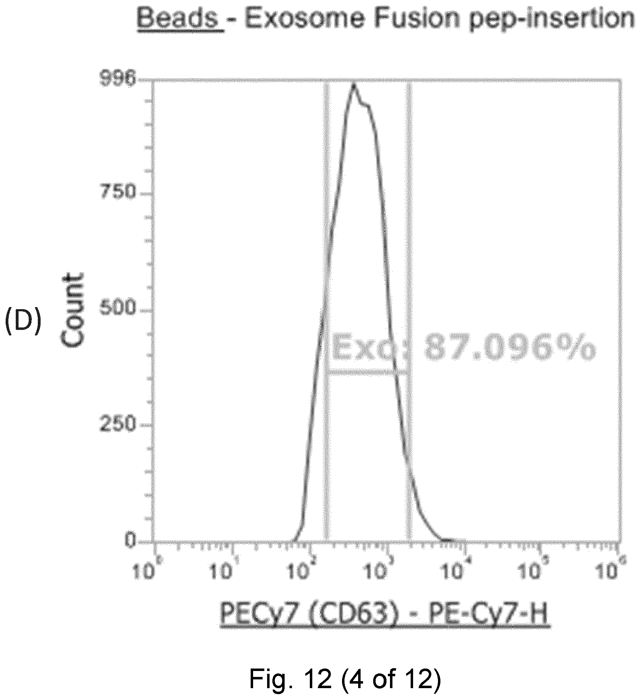

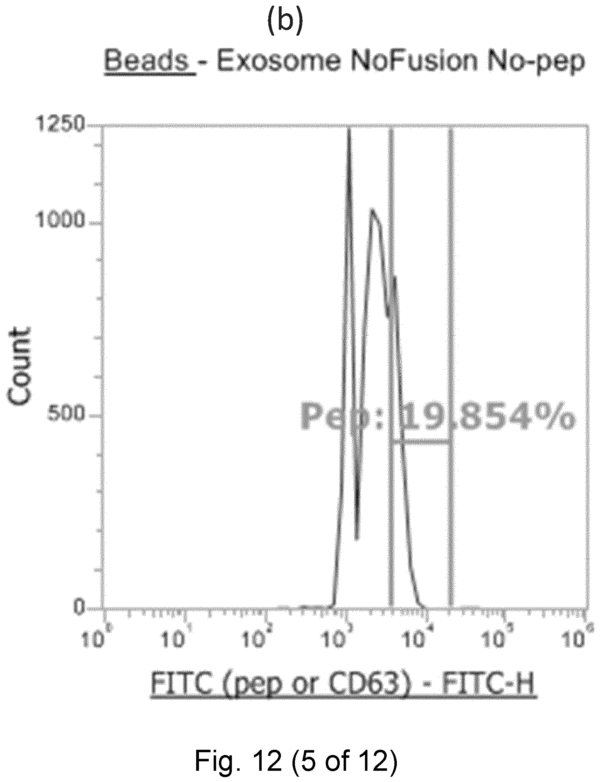

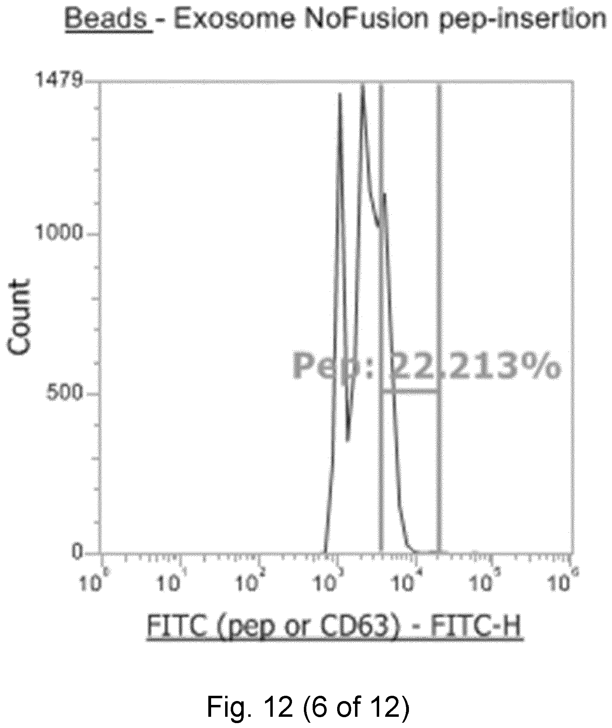

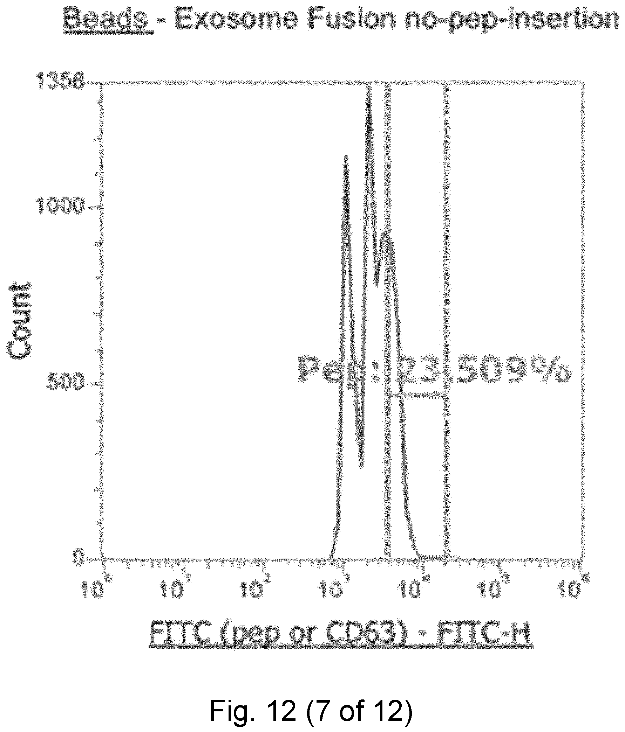

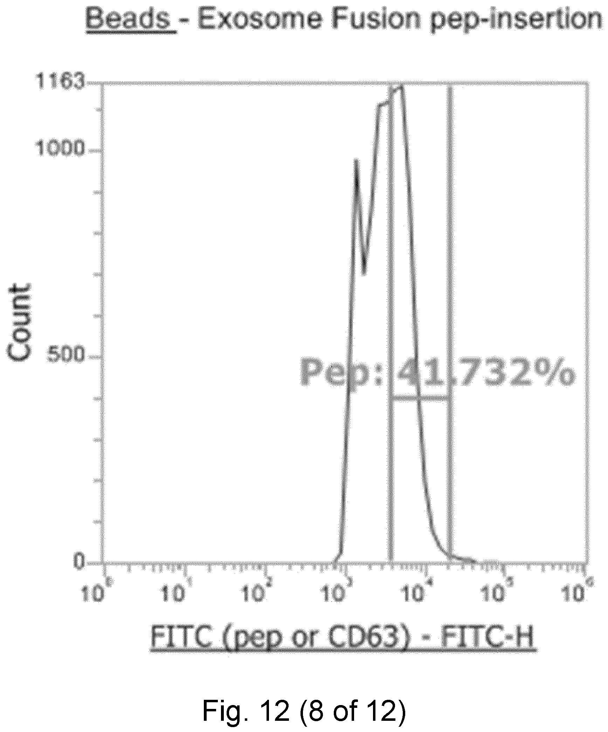

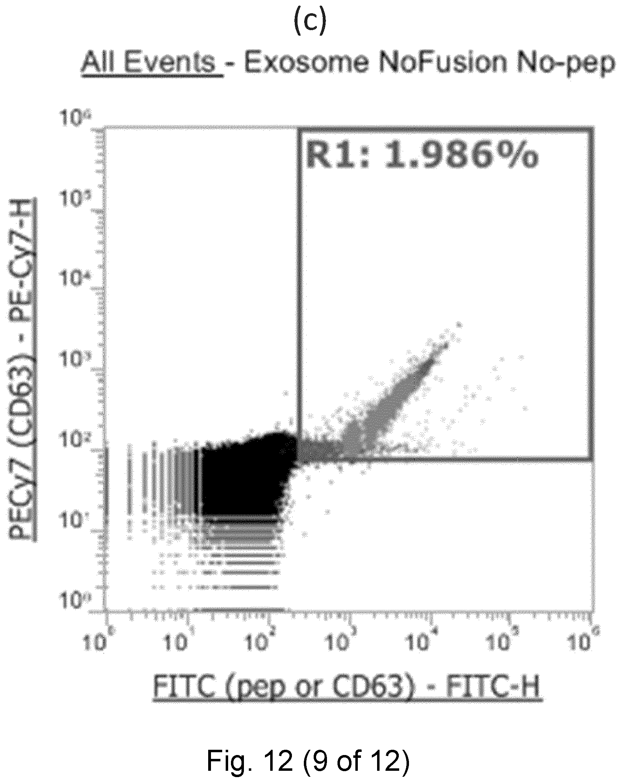

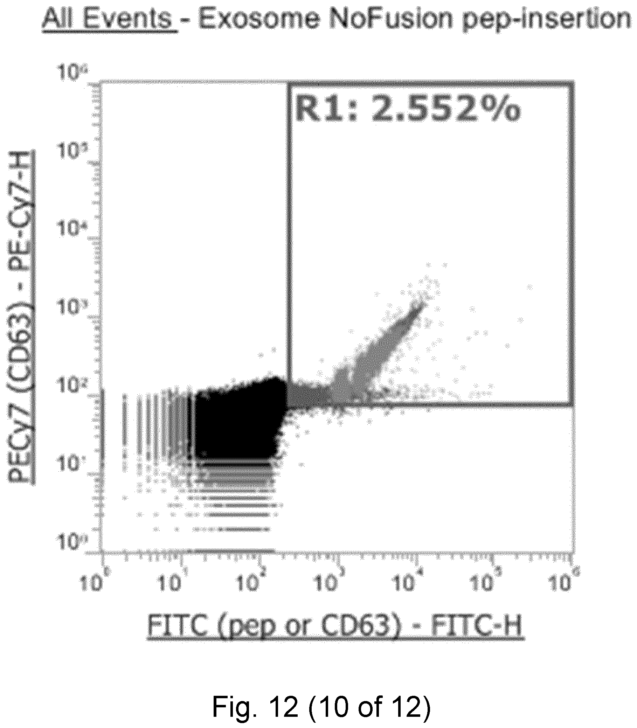

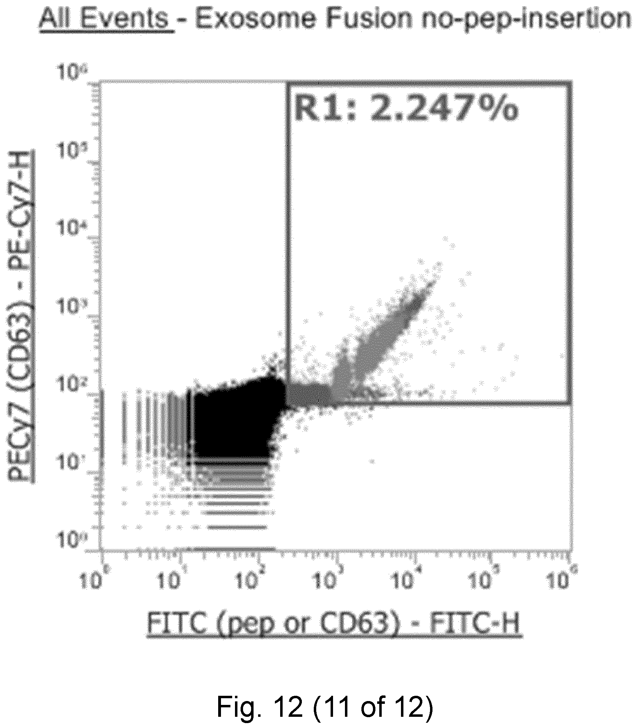

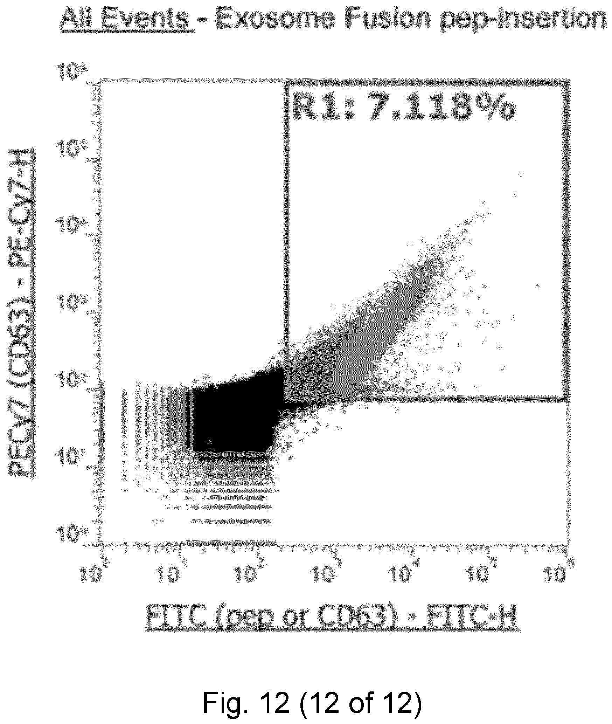

Fig. 12 shows diagrams of the results of flow cytometry of hybrid liposome exosomes of Lipocapsulater FD-S PL to which a peptide shown in SEQ ID NO: 5 was added and an exosome.
[Fig. 13A]
Fig. 13A is a diagram showing the results of an examination of a cell growth inhibitory ability of peptide-binding hybrid liposome exosomes in Experimental Example 8.
[Fig. 13B]
Fig. 13B is a diagram showing the results of an examination of a cell growth inhibitory ability of peptide-binding hybrid liposome exosomes in Experimental Example 8.
[Fig. 13C]
Fig. 13C is a diagram showing the results of an examination of a cell growth inhibitory ability of peptide-binding hybrid liposome exosomes in Experimental Example 8.
[Fig. 13D]
Fig. 13D is a diagram showing the results of an examination of a cell growth inhibitory ability of peptide-binding hybrid liposome exosomes in Experimental Example 8.
[Fig. 13E]
Fig. 13E is a diagram showing the results of an examination of a cell growth inhibitory ability of peptide-binding hybrid liposome exosomes in Experimental Example 8.
[Fig. 14A]
Fig. 14A is a diagram showing the results of an examination of a cell growth inhibitory ability of peptide-binding hybrid liposome exosomes in Example 8.
[Fig. 14B]
Fig. 14B is a diagram showing the results of an examination of a cell growth inhibitory ability of peptide-binding hybrid liposome exosomes in Example 8.
[Fig. 14C]
Fig. 14C is a diagram showing the results of an examination of a cell growth inhibitory ability of peptide-binding hybrid liposome exosomes in Example 8.
[Fig. 14D]
Fig. 14D is a diagram showing the results of an examination of a cell growth inhibitory ability of peptide-binding hybrid liposome exosomes in Example 8.
[Fig. 14E]
Fig. 14E is a diagram showing the results of an examination of a cell growth inhibitory ability of peptide-binding hybrid liposome exosomes in Example 8.
[Fig. 15]

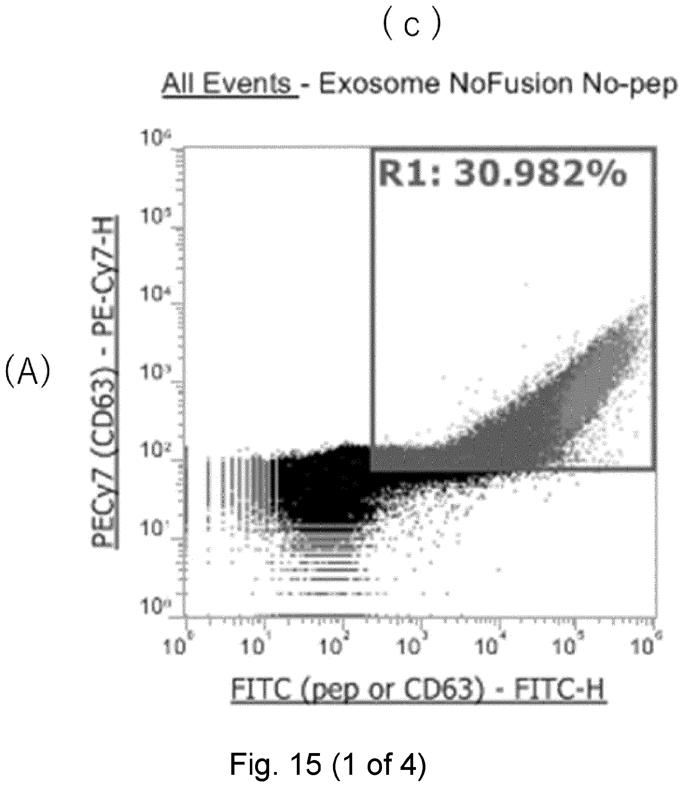

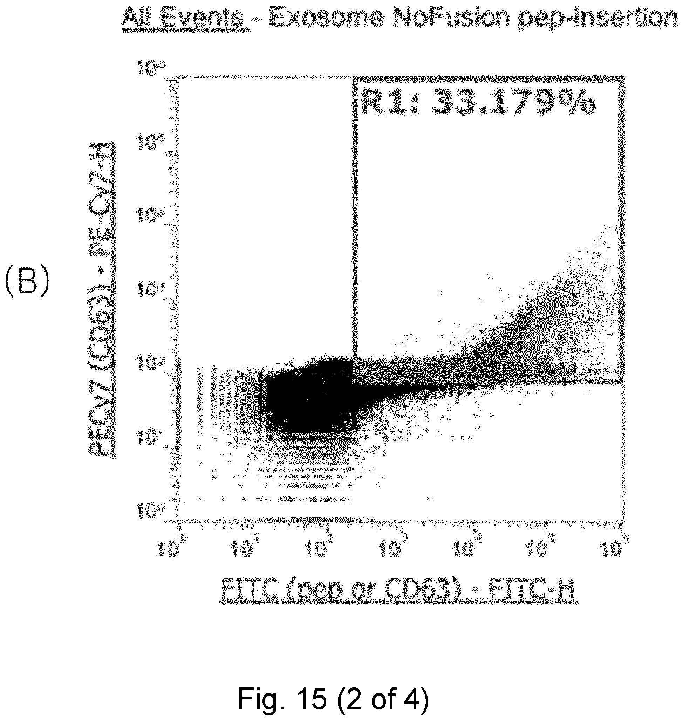

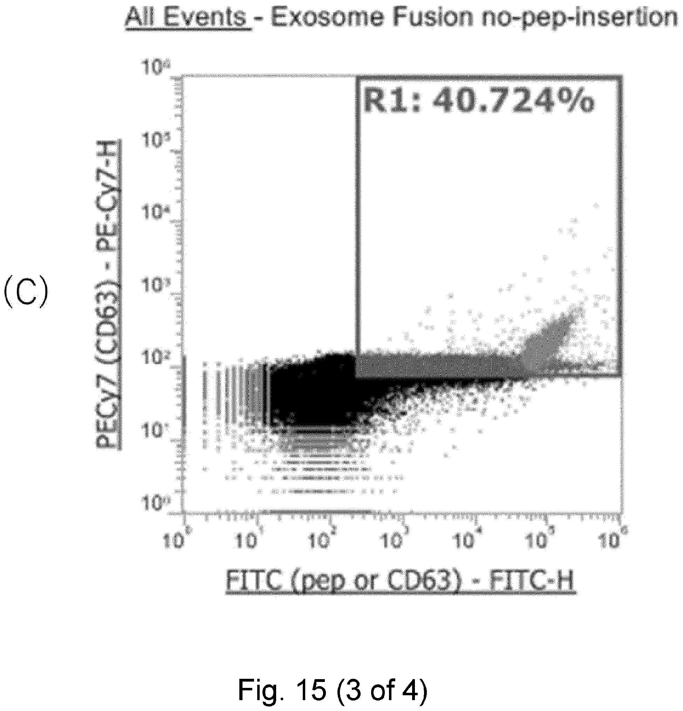

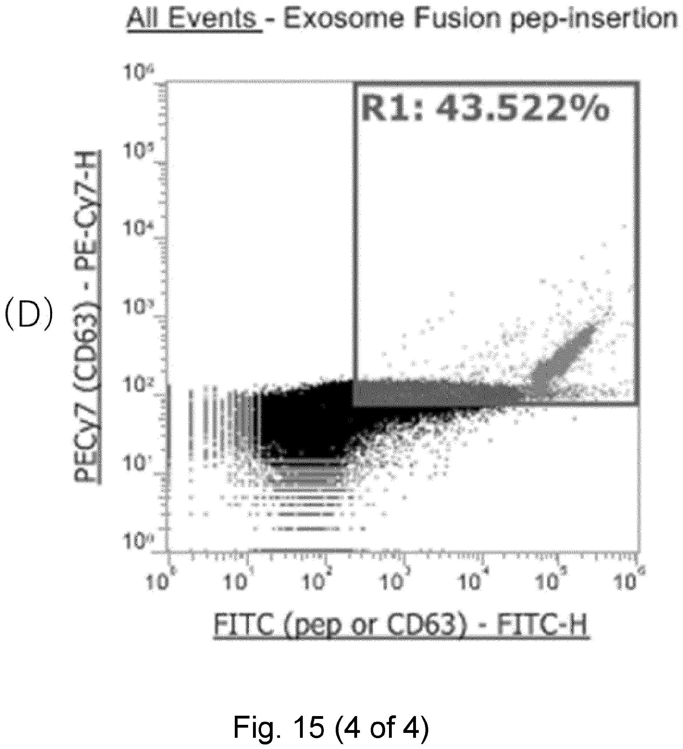

Fig. 15 shows diagrams of the results of flow cytometry of hybrid liposome exosomes of Lipocapsulater FD-U PL to which a peptide shown in SEQ ID NO: 5 was added and an exosome.

[Fig. 16]

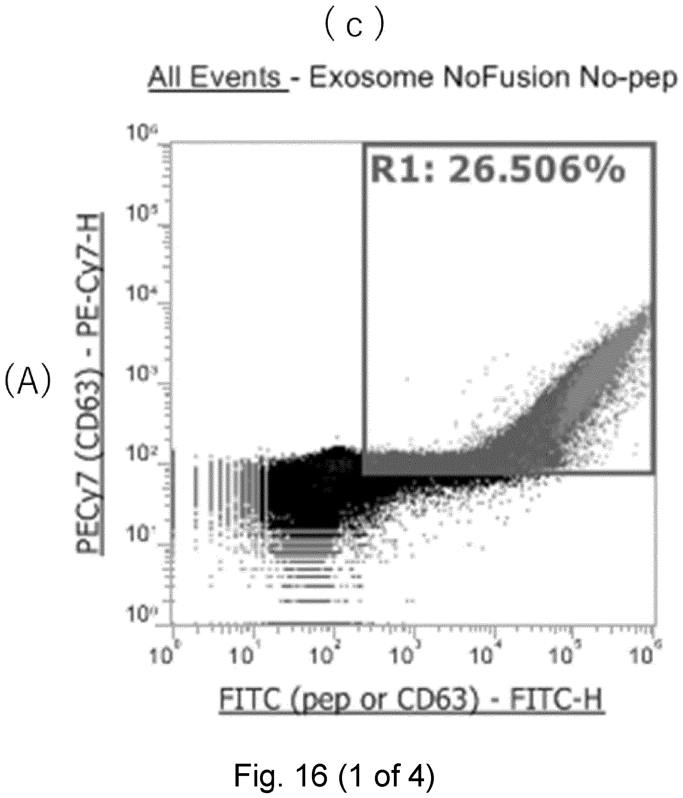

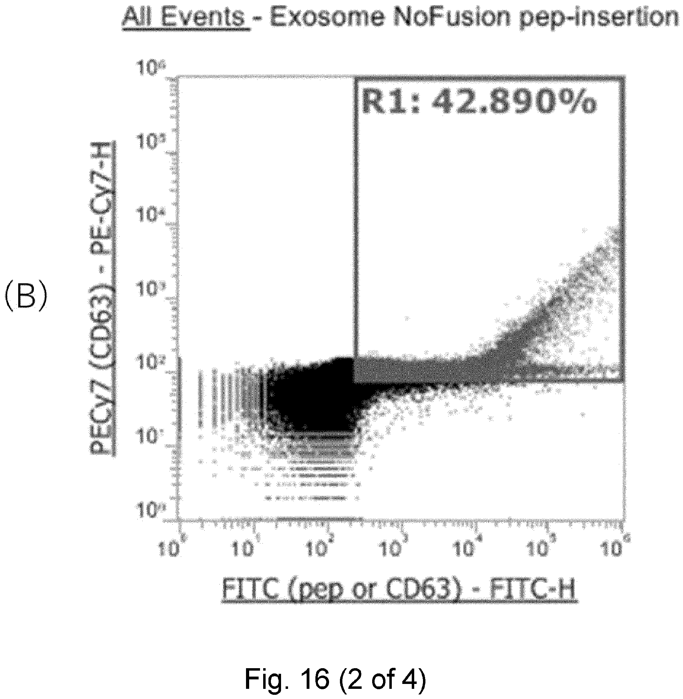

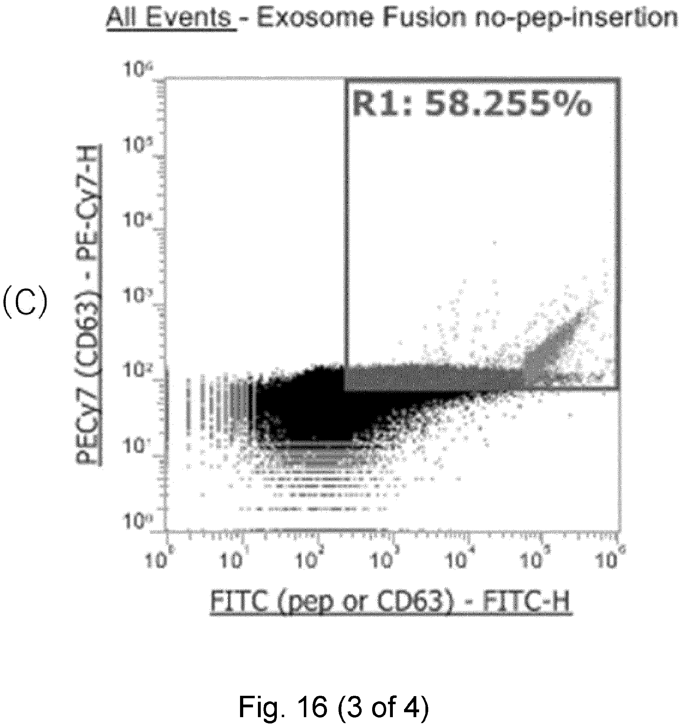

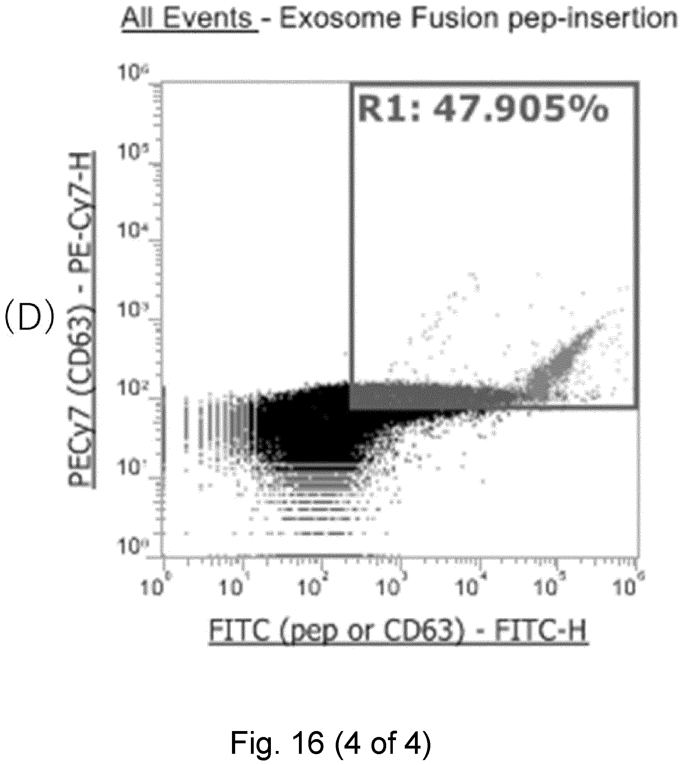

Fig. 16 shows diagrams of the results of flow cytometry of hybrid liposome exosomes of Lipocapsulater FD-U PL to which a peptide shown in SEQ ID NO: 3 was added and an exosome.

DETAILED DESCRIPTION OF THE INVENTION

Hereinafter, forms for implementing the present invention will be described in detail with reference to the drawings. Here, embodiments of the present invention described below are only for examples, and the present invention is not limited to the embodiments described below.

<Peptide-binding hybrid liposome exosome>

A peptide-binding hybrid liposome exosome according to a first embodiment, which is an example of the present invention, comprises in a lipid bilayer an exosome marker protein selected from CD63, CD81, and CD9 and peptide-binding lipid derivatives in which a peptide binds to a hydrophilic part of a complex lipid. Here, the present invention is not limited to the peptide-binding hybrid liposome exosome, and a peptide-binding exosome to which a predetermined peptide binds without the exosome being fused with a liposome also falls within the scope of the present invention.

[Exosome marker protein]

CD63, CD81, and CD9 are membrane proteins that are localized in the lipid bilayer of the exosome and are specifically found in the exosome. A method of forming a peptide-binding hybrid liposome exosome of the present invention is not particularly limited, but the peptide-binding hybrid liposome exosome of the present embodiment is obtained by fusing a predetermined liposome with an exosome. Therefore, the peptide-binding hybrid liposome exosome retains membrane proteins derived from an exosome, and can be clearly distinguished from the exosome and the liposome by labeling an exosome marker protein selected from CD63, CD81, and CD9 and peptides constituting peptide-binding lipid derivatives to be described below together.

(Characteristics of CD63)

CD63 is also known as LAMP-3(Lysosomal-associated membrane protein 3), and is a 30 kDa to 60 kDa lysosome membrane protein belonging to the tetraspanin family that has many important roles in immune-physiological functions. CD63 is known to mediate signal transmission related to regulation of cell development, activation, growth, and motility. Since CD63 is expressed on activated platelets, it has been pointed out that it may function as a platelet activation marker, and CD63 is a lysosome-associated membrane glycoprotein, and is known to translocate to the cell membrane after platelet activation. The amino acid sequence of human CD63 is listed as Accession No. P08962.2 in the sequence information database Genbank provided by the NCBI, and disclosed over the Internet. The content of the web page is incorporated into a part of this specification by reference.

(Characteristics of CD81)

CD81 is a single-chain protein that belongs to the tetraspan family with four transmembrane domains. On the cell membrane, in CD81, both the N-terminal and the C-terminal are located in the cytoplasm, and two peptide loops are exposed outside the cell. CD81 does not contain sugar chains and has a molecular weight of 26 kDa. It has been pointed out that CD81 has a wide tissue distribution and is present in association with other tetraspan family members and the like. Immune B cells express relatively high levels of CD81 throughout their differentiation stages. The amino acid sequence of human CD81 is listed as Accession No. P60033.1 in the sequence information database Genbank provided by the NCBI and disclosed over the Internet. The content of the web page is incorporated into a part of this specification by reference.

(Characteristics of CD9)

CD9 is a single-chain membrane protein having a molecular weight of 24 kDa and belongs to the tetraspan family. The CD9 antigen has four transmembrane domains and has a structure in which both the N-terminal and the C-terminal are present in the cell. It is found that CD9 is present in α granules of platelets, monocytes, pre-B cells, eosinophils, basophils, activated T cells and the like. It is thought that CD9 is associated with molecules such as very late activation (VLA) integrin molecules and HLA-DR, and involved in adhesion between cells, signal transmission, and cell motility. The amino acid sequence of human CD9 is listed as Accession No. P21926.4 in the sequence information database Genbank provided by the NCBI and disclosed over the Internet. The content of the web page is incorporated into a part of this specification by reference.

(Other exosome marker proteins)

Here, in addition to the exosome marker proteins localized in the lipid bilayer of the exosome, the peptide-binding hybrid liposome exosome can also be identified using an exosome marker protein encapsulated in the exosome as a label. As such an exosome marker protein encapsulated in the exosome, for example, Alix may be exemplified.

Alix (ALG-2-interacting protein X) is a protein encoded by the PDCD6IP gene, also known as AIP1 (ALG-2-interacting protein 1) or Hp95. Alix is known to be involved in apoptosis (cell death) through a mechanism of action involving ALG-2(apoptosis linked gene 2 product). Here, ALG-2 is a 22 kDa protein having five repetitive EF-hand structures, and is known as a calcium-induced apoptosis regulator following endoplasmic reticulum (ER) stress. Alix is known to interact with ALG-2 through the C-terminal proline-rich region and be involved in the formation of multi-vesicular bodies. The amino acid sequence of human Alix is listed as Accession No. Q8WUM4.1 in the sequence information database Genbank provided by the NCBI and disclosed over the Internet. The content of the web page is incorporated into a part of this specification by reference.

[Method of obtaining exosome]

In the present embodiment, the exosome is derived from stem cells, and is derived from, more specifically, stem cells selected from the group consisting of pluripotent stem cells, mesenchymal stem cells, ectoderm-derived cells, mesoderm-derived cells, and endoderm-derived cells. Therefore, since the peptide-binding hybrid liposome exosome of the present embodiment can be directly applied to exosomes derived from cells with various properties, targeting to various cells can be imparted to the peptide-binding hybrid liposome exosome depending on properties of the exosome.

As such pluripotent stem cells, mesenchymal stem cells, ectoderm-derived cells, mesoderm-derived cells, and endoderm-derived cells, for example, the following cells may be exemplified.

(Pluripotent stem cells)

Pluripotent stem cells are cells that potentially have an ability to differentiate into various tissues of a living body, and specifically, cells that can differentiate into any of endoderm, mesoderm, and ectoderm. Examples of such cells include embryonic stem cells (ES cells) and induced pluripotent stem cells (iPS cells).

(Mesenchymal stem cells)

Mesenchymal stem cells are stem cells that have an ability to differentiate into tissues mainly derived from mesoderm, tissues derived from some ectoderm and endodermal tissue. As markers for mesenchymal stem cells, adhesive molecules CD106, CD166, and CD29, and CD105, CD73, CD44, CD90, and CD71 are known, and as negative markers, adhesive molecules CD31, CD18, and CD56, hematopoietic markers CD45, CD34, CD14, and CD11, and costimulation molecules CD80, CD86, and CD40 are known.

(Ectoderm-derived cells)

Examples of ectoderm-derived cells include keratinizing epithelial cells (epidermal keratinocytes, epidermal basal cells, nail keratin-producing cells, nail bed basal cells, medullary hair stem cells, cortical hair stem cells, cuticular hair stem cells, cuticular root sheath cells, Huxley layer root sheath cells, Henle layer root sheath cells, outer root sheath cells, and hair follicle cells), stratified epithelial cells (single-layer squamous epithelial cells of the cornea, tongue, oral cavity, esophagus, anal canal, urethra, and vagina; basal cells of the cornea, tongue, oral cavity, esophagus, anal canal, urethra, and vagina; and bladder epidermal cells), inner hair cells of the organ of Corti, outer hair cells of the organ of Corti, basal cells of the olfactory epithelium, cold sensory neurons, thermosensory sensory neurons, Merkel cells of the epidermis, olfactory receptor neurons, pain sensory neurons, retinal photoreceptor cells (rod cells, blue cone cells, green cone cells, red cone cells), deep sensory neurons, tactile sensory neurons, type I glomus cells, type II glomus cells, type I hair cells of the vestibular system, type II hair cells of the vestibular system, type I taste bud cells, autonomic nerve cells (cholinergic neurons, adrenergic neurons, and peptidergic neurons), inner pillar cells of the organ of Corti, outer pillar cells of the organ of Corti, inner phalangeal cells of the organ of Corti, outer phalangeal cells of the organ of Corti, border cells of the organ of Corti, Hensen's cells of the organ of Corti, vestibular organ supporting cells, taste bud supporting cells, olfactory epithelial supporting cells, Schwann cells, satellite cells, enteric glial cells, central nervous system neurons and glial cells (astrocytes, neurons, oligodendrocytes, and spindle neurons), and lens cells (anterior lens epidermal cells, and crystalline lens fiber cells).

(Mesoderm-derived cells)

Examples of mesoderm-derived cells include hepatocytes, adipocytes (white adipocytes, and brown adipocytes), Ito cells, pararenal cells, glomerular epithelial cells, proximal tubular brush border cells, thin segment cells of the loop of Henle, distal tubule cells, collecting tubule cells, type I pneumocyte epithelial cells, centroacinar cells, smooth muscle duct cells (principal cells, and intercalated cells), duct cells, intestinal brush border cells, exocrine striatal duct cells, gallbladder epithelial cells, testicular efferent duct non-ciliated cells, epididymal principal cells, epididymal basal cells, ameloblasts, meniscal epithelial cells, interdental epithelial cells of the organ of Corti, loose connective tissue fibroblasts, corneal fibroblasts, tendon fibroblasts, bone marrow reticular connective tissue fibroblasts, other non-epithelial fibroblasts, pericytes, nucleus pulposus cells of the intervertebral disc, cementoblasts, odontoblasts, hyaline cartilage chondrocytes, fibrocartilage chondrocytes, elastic cartilage chondrocytes, osteoblasts, osteoprogenitor cells, hyalocytes of the vitreous body, stellate cells of the ear, pancreatic stellate cells, contractile cells, skeletal muscle cells (slow muscle cells, fast muscle cells, intermediate muscle cells, nuclear bag fibers of muscle spindles, and nuclear chain fibers of muscle spindles), satellite cells, cardiomyocytes (cardiomyocytes, segmented cardiomyocytes, and Purkinje fiber cells), smooth muscle cells, myoepithelial cells of the iris, myoepithelial cells of the exocrine glands, megakaryocytes, monocytes, connective tissue macrophages, epithelial Langerhans cells, osteoclasts, dendritic cells, microglial cells, neutrophils, eosinophils, basophils, hybridoma cells, mast cells, Th2 cells, regulatory T cells, cytotoxic T cells, natural killer T cells, B cells, natural killer cells, reticulocytes, stem cells and progenitor cells of the blood and immune system, erythroblasts, myelocytes, oocytes, sperm cells, spermatocytes, spermatogenous cells, nurse cells, follicular ovarian cells, Sertoli cells, thymic epithelial cells, interstitial kidney cells, and any cultured cells derived therefrom.

(Endoderm-derived cells)

Examples of endoderm-derived cells include salivary gland mucus cells, salivary gland (n1) cells, von Ebner gland cells of the taste buds, mammary gland cells, lacrimal gland cells, auditory canal gland cells, eccrine glands, glandular dark cells, eccrine gland clear cells, apocrine gland cells, eyelash gland cells, sebaceous gland cells, Bowman's gland cells of the epithelial cells of the nose, Brunner's gland cells of the duodenum, seminal vesicle cells, prostate cells, bulbourethral gland cells, Bartholin gland cells, urethral gland cells, endometrial cells, goblet cells, gastric mucosal cells, principal cells of the stomach, parietal cells, pancreatic acinar cells, paneth cells of the small intestine, type II pneumocytes of the lungs, clara cells of the lungs, anterior pituitary cells (growth hormone-secreting cells, prolactin-secreting cells, pituitary-tropic thyroid cells, gonadotropic cells, and adrenocorticotrophic hormone-secreting cells), melanocyte-stimulating hormone-producing cells, large neurosecretory neurons, thyroid cells (follicular cells, and parafollicular cells), parathyroid cells (principal cells, and acidophilic cells), adrenal cells (chromaffin cells, etc.), Leydig cells, internal capsule cells, luteal cells (granulosa lutein cells, and theca lutein cells), juxtaglomerular cells, macula densa cells, circumpolar cells, mesangial cells, and any cultured cells derived therefrom.

In order to obtain exosomes from these cells, these cells are cultured for 1 day to 30 days in a general medium commonly used for cell culture, the culture solution is collected, as necessary, and exosomes with a particle size of 20 nm to 150 nm (in diameter) can be obtained by separation and concentration by conventionally commonly used column chromatography such as gel filtration chromatography, affinity column chromatography using antibodies against antigens selected from the above CD63, CD81, and CD9, size exclusion chromatography, and phosphatidylserine affinity chromatography or flow cytometry. Here, it is clear that the culture supernatant containing an exosome does not contain any solid component other than the exosome, and if the exosome concentration operation is not required depending on characteristics of an operation to be performed, the above separation and concentration operation may not be performed.

Here, the medium that can be used in cell culture for obtaining exosomes is not particularly limited and any general cell culture medium can be used. However, in order to increase the exosome production ability, for example, it is preferable to use a completely synthetic medium that does not contain an exosome, and when a natural medium is used or even when a naturally derived component such as serum is added to a synthetic medium, it is preferable that an exosome be removed from the natural medium or naturally derived raw materials.

[Peptide-binding lipid derivatives]

The peptide-binding hybrid liposome exosome of the present embodiment comprises in a lipid bilayer peptide-binding lipid derivatives in which a peptide binds to a hydrophilic part of a complex lipid. Although the method of forming a peptide-binding hybrid liposome exosome of the present invention is not particularly limited, the peptide-binding hybrid liposome exosome of the present embodiment is obtained by forming a liposome containing peptide-binding lipid derivatives and fusing the liposome with an exosome. Here, the present embodiment is not limited to an aspect in which peptide-binding lipid derivatives are incorporated into a peptide-binding hybrid liposome exosome by incorporating them into a liposome, but peptide-binding lipid derivatives may be incorporated into an exosome or directly incorporated into a hybrid liposome exosome. Since the peptide-binding lipid derivatives are easily exposed on the outer surface of the peptide-binding hybrid liposome exosome, it is possible to impart an additional function related to incorporation into target cells to the peptide-binding hybrid liposome exosome. Therefore, it is possible to provide a carrier for a drug delivery system that can deliver an active component with a higher concentration to target cells.

(Peptide that binds to peptide-binding lipid derivatives)

In the present embodiment, the peptide that binds to peptide-binding lipid derivatives preferably has 10 to 30 amino acid residues. When the number of amino acid residues of the peptide is within the above range, the function of the peptide that binds to peptide-binding lipid derivatives is sufficiently secured, and a bulky peptide is unlikely to inhibit fusion of the lipid bilayer of the peptide-binding hybrid liposome exosome and the lipid bilayer of the endosome. The peptide may have, for example, 10 to 27, 15 to 20, or 17 to 19 amino acid sequences.

When more specific description is provided in terms of these peptides, the above peptide is preferably a peptide that forms an $\alpha$-helix structure with a hydrophobic surface in an endosome or a peptide having a basic residue.

(Peptide that forms $\alpha$-helix structure with hydrophobic surface)

A peptide that forms an $\alpha$-helix structure with a hydrophobic surface in an endosome generally has a certain repeating sequence with a pitch of 3 to 4 residues as amino acid residues. Such a repeating sequence preferably contains a small bulk hydrophobic amino acid, a small bulk acidic amino acid and/or a polar amino acid. While the present invention is not bound by this theory, particularly, acidic groups contained in the side chains of acidic amino acids have properties as weak acids, and thus the peptide-binding hybrid liposome exosome is incorporated into an endosome, and when the surrounding environment becomes acidic, the groups are protonated and tend to form an $\alpha$-helix structure with a hydrophobic surface together with other hydrophobic amino acid residues. When 10 to 30 amino acid residues having an $\alpha$-helix structure with a hydrophobic surface are exposed on the outer surface of the lipid bilayer, they easily penetrate the lipid bilayer constituting an endosome (lysosome), and the peptide-binding hybrid liposome exosome and the endosome (lysosome) are more likely to be fused. That is, due to the action of the $\alpha$-helix structure with a hydrophobic surface, the peptide-binding hybrid liposome exosome is more susceptible to endosome release, and the contents of the peptide-binding hybrid liposome exosome are more likely to be incorporated into the cytoplasm.

When the peptide is a peptide that forms an $\alpha$-helix structure with a hydrophobic surface, the hydrophobic amino acid residue contained in the repeating sequence is preferably at least one selected from an alanine residue, a leucine residue, and a methionine residue and more preferably at least one selected from an alanine residue and a leucine residue. In addition, at least one residue of amino acid residues constituting the repeating sequence is preferably at least one selected from an acidic amino acid residue and a polar amino acid residue described below. The acidic amino acid residue contained in the repeating sequence is preferably at least one selected from an aspartic acid residue and a glutamic acid residue and more preferably a glutamic acid residue. The polar amino acid residue contained in the repeating sequence is preferably at least one selected from an asparagine residue and a glutamine residue and more preferably a glutamine residue. Here, it is most preferable that the repeating sequence contain a combination of a hydrophobic amino acid residue and an acidic amino acid residue.

As an example of a peptide that satisfies such a condition, for example, an amino acid sequence having 30 residues shown in SEQ ID NO: 1 (WEAALAEALAEALAEHLAEALAEALEALAA) may be exemplified.

(Peptide having basic residue)

Examples of peptides having a basic residue include a peptide having at least one basic amino acid residue. When basic groups of the basic amino acid residues are protonated in the acidic environment in the endosome (lysosome), they electrostatically interact with hydrophilic parts such as phosphate groups that constitute the surface layer of the lipid bilayer, and thus incorporation of the peptide-binding hybrid liposome exosome into cells can be promoted. The peptide preferably has 2 to 5 consecutive basic amino acid residues, and more preferably 3 to 4 consecutive amino acid residues. When the above peptide has such consecutive basic amino acid residues, incorporation of the peptide-binding hybrid liposome exosome into cells can be further promoted.

In addition, the peptide preferably does not contain acidic amino acid residues. Thereby, the interaction between the basic amino acid residue in the peptide and the phosphate group on the surface layer of the lipid bilayer is not inhibited by the acidic amino acid residue, and incorporation of the peptide-binding hybrid liposome exosome into cells can be further promoted.

As an example of peptides that satisfy such conditions, for example, amino acid sequences shown in SEQ ID NO: 2 (LLIILRRRIRKQAHAHSK), SEQ ID NO: 3 (CGRKKRRQRRR), SEQ ID NO: 4 (GIGAVLKVLTTGLPALISWIKRKRQQ), and SEQ ID NO: 5 (RQIKIWFQNRRMKWKK) and having 18 residues, 11 residues, 26 residues, and 16 residues, respectively, may be exemplified.

Here, in the present embodiment, the amino acid residues constituting the peptide are not limited to amino acid residues derived from natural amino acids, and as long as effects of the present invention are not impaired, they may additionally include amino acid residues derived from amino acids other than natural amino acids and amino acid residues derived from amino acids, which are various isomers of natural amino acids such as optical isomers. Only 1 to 3 residues of various isomers of such unnatural amino acids or natural amino acids of the above peptide are preferably contained in the entire peptide, and all amino acid residues are more preferably amino acid residues derived from natural amino acids.

(Complex lipid constituting peptide-binding lipid derivatives)

Examples of complex lipids constituting peptide-binding lipid derivatives include complex lipids known as biologically derived substances that are soluble in nonpolar solvents and constitute the lipid bilayer, and more specifically, complex lipids having, at an end, any reactive group selected from the group consisting of primary amino groups, carboxyl groups, hydroxy groups, phosphate groups, and thiol groups may be exemplified. These complex lipids are selected from, for example, sphingophospholipids, glycosphingolipids, glycerophospholipids, glyceroglycolipids, ether type phospholipids, and ether type glycolipids. Since these complex lipids have polar groups derived from phosphate groups in their molecules, polar groups derived from sugars, and the like, such polar groups can react with polar groups of the peptide, and the complex lipid and the peptide can be easily bonded.

Examples of sphingophospholipids include sphingomyelin, and examples of glycosphingolipids include cerebroside (galactocerebroside, sulfatide, glucocerebroside, etc.), ganglioside, globoside, and sulfatide. In addition, examples of glycerophospholipids include phosphatidic acid, phosphatidylcholine, phosphatidylserine, phosphatidylethanolamine, and phosphatidylinositol, and examples of glyceroglycolipids include condensates of any monosaccharide or oligosaccharide and diacylglycerol such as monogalactosyldiacylglycerol. In addition, for example, saturated aliphatic groups and unsaturated aliphatic groups constituting hydrophobic groups of diacylglycerol moieties constituting glycerophospholipids and glyceroglycolipids are generally saturated aliphatic groups and unsaturated aliphatic groups derived from saturated or unsaturated fatty acids having 12 to 20 carbon atoms. Among these, examples of saturated fatty acids include lauric acid, myristic acid, pentadecylic acid, palmitic acid, heptadecanoic acid, stearic acid, and arachidic acid, and examples of unsaturated fatty acids include mono-unsaturated fatty acids (myristoleic acid, palmitoleic acid, sapienic acid, oleic acid, elaidic acid, vaccenic acid, gadoleic acid, eicosenoic acid, etc.), di-unsaturated fatty acids (linoleic acid, eicosadienoic acid, etc.), tri-unsaturated fatty acids (linolenic acid, pinolenic acid, etc.), tetra-unsaturated fatty acids (stearidonic acid, arachidonic acid, etc.), penta-unsaturated fatty acids (eicosapentaenoic acid, etc.), and hexa-unsaturated fatty acids (hexadocosaenoic acid, etc.). The aliphatic groups constituting the glycerophospholipid and glyceroglycolipid may include a mixture of saturated aliphatic groups and unsaturated aliphatic groups and a mixture of aliphatic groups having different numbers of carbon atoms and aliphatic groups having different numbers of unsaturated groups.

Ether type phospholipids and ether type glycolipids are complex lipids in which one or two long-chain hydrocarbon groups are ether-bonded to glycerol and which have a phosphate group or sugar as a polar group, and are widely found in thermophilic bacteria, and platelet activating factors and the like are known to have a similar structure. In this case, two hydrocarbon groups that bind to glycerol are preferably saturated or unsaturated hydrocarbon groups having 12 to 20 carbon atoms. That is, ether type phospholipids and ether type glycolipids are preferably those having a structure in which one or two molecules of a higher saturated or unsaturated alcohol having 12 to 20 carbon atoms and glycerol are bonded via an ether bond, and a phosphate group, or a monosaccharide or oligosaccharide is bonded to the remaining one hydroxyl group of glycerol.

Details of complex lipids are described in Int. J. Mol. Sci. 2019, 20, 2167, and Nat. Rev. Mol. Cell Biol., 2018, 19, 281, the contents of which are incorporated into a part of this specification by reference.

(Method of forming peptide-binding lipid derivatives)

In the present embodiment, in order to form peptide-binding lipid derivatives, any chemical reaction method in which the N-terminal amino group or C-terminal carboxyl group of the peptide is covalently bonded to a phosphate group, amino group, hydroxyl group or the like of the complex lipid can be used. In the present embodiment, among such chemical reaction methods, a chemical reaction method in which a phosphate group, amino group, hydroxyl group or the like of a complex lipid is reacted with a carboxyl group or amino group introduced into the complex lipid via a linker to be described below and an amino group is preferably used. Examples of such chemical reaction methods include carbodiimide crosslinking reaction, NHS ester crosslinking reaction, and imidoester crosslinking reaction methods. In these chemical reaction methods, a carboxyl group or an amino group is activated with a predetermined compound and this is then covalently bonded to an amino group or a carboxyl group. Crosslinking agents for implementing these chemical reaction methods are commercially available from Thermo Fisher Scientific Inc., and those skilled in the art can use such commercially available products and implement the above chemical reaction method.

Examples of linkers for introducing a carboxyl group or amino group into a complex lipid include polyalkylene glycols such as polyethylene glycol and polypropylene glycol, and compounds in which a primary amino group or carboxyl group is introduced into these polyalkylene glycols. In this case, the polymerization degree of ethylene glycol or propylene glycol includes, for example, a polymerization degree of 20 or more and 50 or less, or 30 or more and 45 or less. More specific examples of linkers include compounds such as HOOC-PEG-COOH, $H_2$N-PEG-COOH, and $H_2$N-COOH-$NH_2$. These linkers are commercially available from Thermo Fisher Scientific Inc., and those skilled in the art can appropriately use such commercially available products. In addition, peptide-binding lipid derivatives may be formed using complex lipid derivatives obtained by introducing the crosslinking agent into the complex lipid (commercially available from Avanti Polar Lipids, Inc.) via the linker.

[Method of detecting peptide-binding hybrid liposome exosome]

When a peptide-binding hybrid liposome exosome in which antibodies conjugated with a first fluorescent dye, which specifically bind to an exosome marker protein selected from CD63, CD81, and CD9 and antibodies conjugated with a second fluorescent dye, which specifically bind to any of peptides constituting peptide-binding lipid derivatives are added is analyzed using flow cytometry, the peptide-binding hybrid liposome exosome of the present invention can be detected as vesicles in which the antibodies conjugated with the first fluorescent dye and the antibodies conjugated with the second fluorescent dye are bonded together.

(Method of producing antibodies against exosome marker proteins and peptides constituting peptide-binding lipid derivatives)

Antibodies against exosome marker proteins and peptides constituting peptide-binding lipid derivatives can be produced using conventionally known methods. These antibodies may be monoclonal antibodies or polyclonal antibodies. In addition, these antibodies may be animal antibodies commonly used in production of antibodies such as mouse antibodies, rat antibodies, guinea pig antibodies, rabbit antibodies, and goat antibodies, or chimeric antibodies thereof. Hereinafter, a method of immunizing rabbits using synthetic peptides to acquire polyclonal antibodies will be exemplified.

(Synthesis of peptide)

Peptides can be synthesized using methods that are commonly used for peptide synthesis, and are generally synthesized by a solid phase method, and the Boc method, Fmoc method and the like in which Boc, Fmoc and the like are used as a protecting group are commonly used. When peptides are synthesized by these methods, final deprotected peptide chains are purified and the purity thereof is checked by high performance liquid chromatography (HPLC), and the molecular weight thereof is checked by a chemical synthesis method, and thus it is checked whether they are appropriately synthesized and purified. The obtained peptides may be lyophilized and stored. Here, particularly, when short-chain peptides are produced as antigens, it is general to synthesize them using the chemical synthesis method, but when higher-molecular-weight proteins are used as antigens, for example, the proteins may be provided as antigens by overexpressing them in cultured cells or the like according to a general method, and purifying them using an appropriate combination of methods commonly used as peptide separation and purification methods such as SDS polyacrylamide gel electrophoresis (SDS-PAGE), gel filtration chromatography, HPLC, and affinity chromatography.

(Rabbit immunization and antibody acquisition)

When the synthesized antigens are short-chain peptides, they are bonded to carrier proteins in order to improve immunogenicity thereof. Typical examples of carrier proteins include keyhole limpet hemocyanin (KLH) and bovine serum albumin (BSA), and the proteins can be bonded to the antigens using a commonly used method such as a maleimide method. Here, when immunogen proteins are polymers, binding to carrier proteins is not always necessary. These conjugated antigens or unconjugated antigens are mixed with an adjuvant and rabbits are immunized. As adjuvants, precipitating adjuvants (sodium hydroxide, aluminum hydroxide, calcium phosphate, aluminum phosphate, alum, PEPeS, carboxyvinyl polymer, etc.), oil-based adjuvants (liquid paraffin, lanolin, Freund, Freund's incomplete adjuvant, and Freund's complete adjuvant) and the like can be used.

When conjugated antigens or unconjugated antigens are administered to rabbits, 0.1 mg to 0.5 mg of the antigens are administered 4 to 6 times at 1 to 4-week intervals. Then, the antibody titer of the antiserum is checked, and when the antibody titer is 0.5 or more, whole blood is collected from the rabbit's carotid artery, and blood cell components are separated to obtain the antiserum. For this antiserum, a commonly used production method such as affinity chromatography using peptides or proteins as antigens is applied and monoclonal antibodies are obtained.

(Fluorescent dye)

The polyclonal antibodies obtained as described above can be labeled with a fluorescent dye. Examples of such fluorescent dyes include commonly used fluorescent dyes with which antibodies are conjugated. Specific examples include Cy3, Cy5, FITC, TRITC, Rhodamine, TAMRA, Alexa Fluor, Texas Red, FAM, APC, PE, ATTO, and DyLight, but the present invention is not limited thereto.

(Peptide-binding hybrid liposome exosome-containing composition)

In the present embodiment, a peptide-binding hybrid liposome exosome-containing composition containing a peptide-binding hybrid liposome exosome is obtained by adding first primary antibodies that specifically bind to an exosome marker protein selected from CD63, CD81, and CD9, second primary antibodies that specifically bind to any of the above peptides (for example, peptides having amino acid sequences shown in SEQ ID NOs: 1 to 5), first secondary antibodies conjugated with a first fluorescent dye, which specifically bind to the first primary antibodies, and second secondary antibodies conjugated with a second fluorescent dye, which specifically bind to the second primary antibodies, and here, the ratio of [the product of the amount of the first primary antibodies added and the antibody titer] and [the product of the amount of the second secondary antibodies added and the antibody titer] is 1:1, the amount ratio of the first primary antibodies and the first secondary antibodies added is 2:1, and the amount ratio of the second primary antibodies and the second secondary antibodies added is 2:1, and when a peptide-binding hybrid liposome exosome that binds to a magnetic bead that specifically binds to a complex lipid contained in an exosome is analyzed using flow cytometry, it is preferable to satisfy the following condition.

$$0.6 \leq A/(A+B+C) \leq 1.0 \quad \text{Formula (1)}$$

(where, in Formula (1), A is an area of a peak indicating the peptide-binding hybrid liposome exosome labeled with both the first fluorescent dye and the second fluorescent dye, B is an area of a peak indicating the exosome labeled with only the first fluorescent dye, and C is an area of a peak indicating the liposome labeled with only the second fluorescent dye).

In addition, the peptide-binding hybrid liposome exosome-containing composition the peptide-binding hybrid liposome exosome of the present embodiment is obtained by fusing an exosome obtained from a cell culture solution with a liposome into which the peptide is introduced, and from the resulting composition, a method of selecting a peptide-binding hybrid liposome exosome-containing composition comprises adding first primary antibodies that specifically bind to an exosome marker protein selected from CD63, CD81, and CD9, second primary antibodies that specifically bind to any of the above peptides (for example, peptides having amino acid sequences shown in SEQ ID NOs: 1 to 5), first secondary antibodies conjugated with a first fluorescent dye, which specifically bind to the first primary antibodies, and second secondary antibodies conjugated with a second fluorescent dye, which specifically bind to the second primary antibodies, where the ratio of [the product of the amount of the first primary antibodies added and the antibody titer] and [the product of the amount of the second secondary antibodies added and the antibody titer] is 1:1, the amount ratio of the first primary antibodies and the first secondary antibodies added is 2:1, and the amount ratio of the second primary antibodies and the second secondary antibodies added is 2:1, and selecting a fraction that satisfies the following condition when a peptide-binding hybrid liposome exosome that binds to a magnetic bead that specifically binds to a complex lipid contained in an exosome is analyzed using flow cytometry.

$$0.6 \leq A/(A+B+C) \leq 1.0 \quad \text{Formula (1)}$$

(where, in Formula (1), A is an area of a peak indicating the peptide-binding hybrid liposome exosome labeled with both the first fluorescent dye and the second fluorescent dye, B is an area of a peak indicating the exosome labeled with only the first fluorescent dye, and C is an area of a peak indicating the liposome labeled with only the second fluorescent dye).

Here, when the above flow cytometry is performed, the following points should be noted.

(a) The antibody titer is determined using a microplate reader such as Synergy HTX (commercially available from BioTek Instruments) by measuring the change in absorbance due to color development by the enzyme using a conventionally known ELISA method (for example, refer to http://www.tkcraft.co.jp/page/elisa.htm).

(b) Mouse antibodies are used as the first primary antibodies, and PE-CY7-labeled anti-mouse IgG goat antibodies are used as the first secondary antibodies. Rabbit antibodies are used as the second primary antibodies, and FITC-labeled anti-rabbit IgG goat antibodies are used as the second secondary antibodies.

(c) Regarding the amount of the first primary antibodies and the second primary antibodies added, within a range of 0.1 mg to 1 mg with respect to 100 μL of the peptide-binding hybrid liposome exosome-containing composition, the fluorescence intensity is measured by changing the addition amount, and the amount of both the first primary antibodies and the second primary antibodies added is determined by determining an addition amount at which a nonspecific fluorescence is not observed under addition amount conditions in which a specific fluorescence changes depending on the addition amount.

(d) As the flow cytometer, Attune (registered trademark) Acoustic Focusing Flow Cytometer (commercially available from Thermo Fisher Scientific Inc.) is used.

Regarding the above flow cytometry using magnetic beads, a PS Capture exosome flow cytometry kit (commercially available from FUJIFILM Wako Pure Chemical Corporation) can be used, and details of some protocols utilizing this kit are described in Nakai, W. et al., A novel affinity-based method for the isolation of highly purified extracellular vesicles. Sci. Rep. 6, 33935, the content of which is incorporated into a part of this specification by reference, but if there is any part that contradicts the description of a specific flow to be described below, the description of a specific flow to be described below has priority.

The principle of the PS Capture Exosome Flow Cytometry Kit is shown in Fig. 3 (a conceptual diagram of exosome flow cytometry using a phosphatidylserine-binding protein Tim4), and in this kit, a protein 2 (Tim4), which has an ability to bind to phosphatidylserine, binds to a magnetic bead 1, and this binds to a calcium ion 3-dependent exosome 4. When an exosome 5 is stained with a fluorescence-labeled antibody 5 specific to the exosome 4 bound to the magnetic bead 1, a flow cytometry peak (y) specific to the exosome 4 bound to the magnetic bead 1 is observed. Here, since binding of Tim4 to phosphatidylserine is dependent on the calcium ion 3, the calcium ion is removed using a chelating agent, and thus the exosome 4 is released from the magnetic bead 1.

More specifically, the following flow is used.

(Separation of peptide-binding hybrid liposome exosome)

(A1) A supernatant is collected by centrifuging a cell culture supernatant at 300×g, 4°C, 5 minutes; 1,200×g, 4°C, 20 minutes; and 10,000×g, 4°C, 30 minutes.

(A2) 0.5 mL of Washing Buffer (10×) (included in the kit) and 4.5 mL of ultra-pure water are added to a 15 mL centrifuge tube and mixed with a vortex mixer. 50 μL (1/100 volume) of Exosome Binding Enhancer (included in the kit) is added thereto and mixed with a vortex mixer.

(A3) 300 μL of 2. is added to a new 1.5 mL microtube.

(A4) Exosome Capture Beads included in the PS Capture Exosome Flow Cytometry Kit are stirred with a vortex mixer for 10 seconds or longer.

(A5) 30 μL of Exosome Capture Beads are collected from (A4) and added to (3).

(A6) The 1.5 mL microtube of (A5) is vortexed for about 5 seconds, spin-downed in a desktop centrifuge and then set on a magnetic stand, and left for about 1 minute, and the supernatant is removed with a pipette.

(A7) 300 μL of (A2) is added to the 1.5 mL microtube of (A6) again, and (6) is repeated.

(A8) 100 μL of the supernatant of (A1) is added to the 1.5 mL microtube of (A7) and vortexed for about 5 seconds. 1 μL (1/100 volume) of Exosome Binding Enhancer is added thereto, and the mixture is vortexed for about 5 seconds.

(A9) (A8) is left at room temperature for 1 hour. During this time, the 1.5 mL microtube is vortexed for about 5 seconds at 20 minutes, 40 minutes, and 1 hour after a sample is added.

(A10) The 1.5 mL microtube of (A9) is spin-downed in a desktop centrifuge and then set on a magnetic stand, and left for about 1 minute, and the supernatant is removed with a pipette.

(A11) 300 μL of (A2) is added to the 1.5 mL microtube of (A10), and (A6) is repeated. This is repeated one more time.

(A12) 300 μL of (A2) is added to the 1.5 mL microtube of (A11) and vortexed for about 5 seconds.

(Fluorescent antibodies staining and flow cytometry)

(B1) 100 μL of the peptide-binding hybrid liposome exosome composition obtained in (A12) is dispensed into each of two 1.5 mL microtubes.

(B2) First primary antibodies and second primary antibodies are added in the addition amounts determined in the above (c) to the 1.5 mL microtube of (B1) and vortexed for about 3 seconds.

(B3) The 1.5 mL microtube of (B2) is left at room temperature for 1 hour. During this time, the 1.5 mL microtube is vortexed for about 5 seconds at 20 minutes, 40 minutes, and 1 hour after first primary antibodies and second primary antibodies are added.

(B4) The 1.5 mL microtube of (B3) is spin-downed in a desktop centrifuge and then set on a magnetic stand and left for about 1 minute, and the supernatant is removed with a pipette.

(B5) 300 μL of (A2) is added to the 1.5 mL microtube of (B4), and (B4) is repeated. This is repeated one more time.

(B6) (B2) to (B5) are repeated for first secondary antibodies and second secondary antibodies.

(B7) 300 μL of (A2) is added to the 1.5 mL microtube of (B6) and vortexed for about 5 seconds.

(B8) The sample of (B7) is used for flow cytometry. When flow cytometry is performed, fractions in which magnetic beads are not aggregated are gated based on a forward scattering and side scattering plot diagram, and the fluorescence intensity of magnetic beads contained in the gate is compared.

In the peptide-binding hybrid liposome exosome-containing composition which satisfies the above conditions or is selected by the above method of selecting a peptide-binding hybrid liposome exosome-containing composition, since most of the vesicles contained are peptide-binding hybrid liposome exosomes, the composition sufficiently exhibits the effect of peptide-binding hybrid liposome exosomes. That is, such a peptide-binding hybrid liposome exosome contained in such a peptide-binding hybrid liposome exosome-containing composition has an additional function related to incorporation into target cells, and can deliver an active component with a higher concentration into target cells.

Here, in the present embodiment, after a peptide-binding hybrid liposome exosome-containing composition is prepared by a method of forming a peptide-binding hybrid liposome exosome to be described below, a peptide-binding hybrid liposome exosome may be selected using the method using the flow cytometry.

<Method of forming peptide-binding hybrid liposome exosome>

A second embodiment, which is an example of the present invention, relates to a method of forming a peptide-binding hybrid liposome exosome. Fig. 1 is a diagram illustrating an outline of a method of forming a peptide-binding hybrid liposome exosome according to the embodiment. The method of forming a peptide-binding hybrid liposome exosome of the present embodiment comprises a step of binding a peptide to a hydrophilic part of a complex lipid to form peptide-binding lipid derivatives, a step of mixing a phospholipid composition containing a phospholipid and the peptide-binding lipid derivatives to form a liposome into which the peptide-binding lipid derivatives are incorporated (A in Fig. 1), and a step of fusing an exosome (B in Fig. 1) with the liposome (C in Fig. 1) to form a peptide-binding hybrid liposome exosome (D in Fig. 1). According to the method of forming a peptide-binding hybrid liposome exosome of the present embodiment, the peptide-binding lipid derivatives can be easily introduced into the peptide-binding hybrid liposome exosome without applying a strong load, and an active component can be easily encapsulated when the liposome is formed. Here, in the following description of the second embodiment, some descriptions that overlap the descriptions in the first embodiment may be omitted.

(Formation of liposome)

When a liposome is formed, for example, commercially available liposome preparation kits can be used. Specifically, a liposome can be formed by dissolving and mixing lyophilized liposomes such as Lipocapsulater FD-S PE, FD-S MA, FD-S PL, FD-U PE, FD-U PL, and the like (commercially available from Katayama Chemical Industries) in a PBS buffer solution or the like at a concentration of 0.1 mg/40 μl to 10 mg/40 μl, and in this case, when the above peptide-binding lipid derivatives are mixed and heating is performed at a temperature equal to or higher than a phase transition temperature of the complex lipid, the peptide-binding lipid derivatives are incorporated into the liposome. Here, in this case, when an active component such as a drug, a nucleic acid, and a peptide is added and mixed, it is also possible to prepare a liposome encapsulating the active component. Here, the scope of the present invention is not limited to an aspect in which the above peptide-binding lipid derivatives are incorporated into a hybrid liposome exosome to be described below by incorporating them into a liposome but encompasses an aspect in which a peptide-binding exosome is formed by mixing an exosome and peptide-binding lipid derivatives and performing necessary processing.

(Fusing of liposome into which peptide-binding lipid derivatives are incorporated with exosome)

Fusion of a liposome into which peptide-binding lipid derivatives are incorporated with an exosome can be performed using, for example, the method described in PTL 1, the content of which is incorporated into a part of this specification by reference. More specifically, a liposome into which peptide-binding lipid derivatives are incorporated and an exosome may be mixed in the presence of polyethylene glycol (PEG). The amount of PEG added may be a concentration of about 0 mass% or more and 40 weight% or less in a mixed solution containing a liposome and an exosome. Here, examples of PEG to be used include PEG500 to PEG10000, and preferably PEG1000 to PEG8000.

<Peptide-binding hybrid liposome exosome>

A peptide-binding exosome according to a third embodiment, which is an example of the present invention, comprises in a lipid bilayer an exosome marker protein selected from CD63, CD81, and CD9 and peptide-binding lipid derivatives in which a peptide binds to a hydrophilic part of a complex lipid. Since the peptide-binding exosome of the present embodiment has the same structure and characteristics as the peptide-binding hybrid liposome exosome of the first embodiment except that it is not fused with a liposome, in the present embodiment, descriptions common to those in the first embodiment will be omitted.

Since the peptide-binding exosome of the present embodiment does not undergo a process of fusion with a liposome, during use in a DDS, when an active component to be delivered to target cells/target tissues is encapsulated into the peptide-binding exosome, it is necessary to use a method different from that of the first embodiment.

In the present embodiment, the peptide-binding exosome can encapsulate low-molecular-weight compounds, polynucleotides, polypeptides and proteins, and for example, when low-molecular-weight compounds are encapsulated into peptide-binding exosomes, a synthase for the low-molecular-weight compounds may be overexpressed in cells that supply exosomes by a conventionally known method such as transformation, transport membrane proteins of the low-molecular-weight compounds are overexpressed in the cells, or as necessary, they are enriched on the exosome membrane, and the low-molecular-weight compounds added from the outside may be incorporated into the cells or exosomes. In addition, when polynucleotides, polypeptides, and proteins are encapsulated into exosomes, the polynucleotides, polypeptides, and proteins may be expressed in cells that supply exosome after an exosome transfer signal is applied as necessary. These methods are widely and generally known to those skilled in the art, and those skilled in the art can use these methods in appropriate combination. Here, the transport mechanism of various biomolecules specific to exosomes is described in known literature such as Journal of Japanese Biochemical Society 91(4): 514-518 (2019), the description of the literature is incorporated into a part of this specification by reference.

EXAMPLES

Hereinafter, examples of the present invention will be described in detail with reference to the drawings. Here, examples shown below are only for examples, and the present invention is not limited to embodiments to be described below.

<Experimental Example 1: examination of cell growth inhibitory effect of peptide-binding to anticancer drug-encapsulated liposome>

Peptide-binding complex lipid derivatives, which are phosphatidylethanolamine bound with a peptide having an amino acid sequence shown in SEQ ID NO: 1, were introduced into a liposome encapsulating doxorubicin, which is an anthracycline-based antitumor antibiotic, and an experiment for verifying cell growth inhibitory ability was performed. Here, when the peptide having an amino acid sequence shown in SEQ ID NO: 1 and phosphatidylethanolamine were bonded, an NHS ester crosslinking reaction was used, and HOOC-PEG-COOH was used as a linker interposed between the peptide and the phosphatidylethanolamine.

[Preparation of HeG2 cells]

50 $\mu$l of ReproCoat (culture container coating agent, commercially available from REPROCELL Inc.) was added to a 96-well microplate. This was left at 37°C, 5% $CO_2$ for 1 hour. HepG2 cells (cultured cells derived from hepatoma cells) that were subcultured and maintained in a T25 flask were detached and collected using a trypsin method. The number of cells was counted and dilution was performed to prepare a $6 \times 10^5$ cells/12 ml solution. 100 $\mu$l of this solution was seeded in a 96-well microplate at $2.5 \times 10^3$ cells/100 $\mu$l/well. Culturing was performed for 24 hours, and adhesion and growth of HepG2 on the bottom surface were checked.

[Preparation of peptide-binding liposome encapsulating doxorubicin]

A solution of peptide-binding complex lipid derivatives was prepared so that a serum-free Dulbecco's Modified Eagle Medium (DMEM medium) contained 50 $\mu$g/ml of peptide-binding complex lipid derivatives (phosphatidylethanolamine to which a peptide having an amino acid sequence shown in SEQ ID NO: 1 was bonded via a linker). A liposome encapsulating doxorubicin (Liposomal Doxorubicin HCl, commercially available FormuMax Scientific Inc.) was added thereto and mixed so that the liposome concentration was 20 $\mu$M ("liposome-peptide mixed solution"). A 10% Lipofectamine (registered trademark) solution (solution for transfection, commercially available from Thermo Fisher Scientific Inc.) was prepared ("10% Lipofectamine (registered trademark) solution") in a 1.5 ml Eppendorf tube. The liposome-peptide mixed solution and the 10% Lipofectamine (registered trademark) solution were mixed at a volume ratio of 1:1 and left at room temperature for 20 minutes. This was mixed with a FBS mixed-DMEM-Glc medium to adjust the sample medium so that the final concentration was 1% for FBS, and 1% for Lipofectamine (registered trademark). The medium in the 96-well microplate was removed using an 8-channel pipette. Using the 8-channel pipette, washing was performed twice with 100 µl of a serum-free DMEM medium (H-Glc). The sample medium was added at 100 µl/well for each group n=8. The sample medium was added 72 hours after HepG2 cells were seeded in the 96-well microplate.

[Growth ability test]

After the sample medium was added, culturing was performed for 48 hours, and using the 8-channel pipette, washing was performed twice with 100 µl of a serum-free DMEM medium (H-Glc). A 10% FBS (exosome-depleted) DMEM (H-Glc) medium was added at 100 µl/well. In addition, after culturing was performed for 48 hours, Premix WST-1 Cell Proliferation Assay System (reagent for cell growth measurement, commercially available from Takara Bio Inc.) was added at 10 µl/well, and color development was performed by performing incubation for 2 hours under 37.5% $CO_2$ conditions. The absorbance at 440 nm was measured using "Synergy HTX Multimode Plate Reader" (culture plate absorptiometer, commercially available from Bio Tek Instruments, Inc.). The average absorbance of the control group (no liposome added) was set as 100, the percentage of the absorbance of each well was calculated and used as an index of the degree of cell growth. Here, the Tukey Kramer method was used for the multiple comparison test.

[Results]

The above results are shown in Table 1 and Fig. 4. As can be clearly understood from the results of Table 1 and Fig. 4, compared to the control group to which no liposome was added, inhibition of cell growth was confirmed in all groups in which doxorubicin was added. However, comparing a "no additive" group in which only doxorubicin was added during liposome preparation and a "peptide+Lipofectamine (registered trademark) 1%" group in which peptide-binding complex lipid derivatives derived from a peptide having an amino acid sequence shown in SEQ ID NO: 1 were added to doxorubicin, even when GALA Peptide was added, the growth inhibitory effect was not enhanced.

[Table 1]

|  | % with respect to control group | standard error |
|---|---|---|
| control group | 100.0 | 3.1 |
| DOX liposome **20 µM** |  |  |
| no additive | 46.9 | 4.3 |
| Lipofection 1% | 75.5 | 7.0 |
| peptide | 46.4 | 3.7 |
| peptide + Lipofection 1% | 71.1 | 6.5 |

<Experimental Example 2: verification of growth inhibitory effect of HepG2 cells by siRNA-encapsulated liposome>

In Experimental Example 1, an initial examination was performed using a doxorubicin-encapsulated liposome, which is an anticancer drug, but for use in a performance evaluation scheme for the peptide-binding hybrid liposome exosome, which is an example of the present invention, an attempt was made to establish an experimental system using a nucleic acid drug whose usage was examined in one embodiment of the present invention.

In Experimental Example 2, siRNA for inhibiting growth of HepG2 cells was selected. Three proteins (VDAC1, SP1, and IGF2BP1) that have been reported to influence growth of HepG2 cells were selected, and siRNA that inhibits expression of these proteins was designed. Therefore, siRNA was expected to inhibit growth of HepG2. Here, for siRNA, siRNA synthesized by Merck KGaA was used.

[Preparation and addition of siRNA sample for inhibiting growth of HepG2 cells]

A sample siRNA solution was prepared according to the Lipofectamine (registered trademark) RNAi MAX protocol. That is, 1 µl of siRNA prepared at a concentration of 10 pmol/ µl and 0.8 µl of PLUS Reagent (transfection promoting reagent, commercially available from Thermo Fisher Scientific Inc.) were mixed in 100 $\mu$l of a serum-free medium. In addition, 2.4 $\mu$L of Lipofectamine (registered trademark) RNAi MAX (transfection reagent, commercially available from Thermo Fisher Scientific Inc.) was mixed and left at room temperature for 25 minutes. siRNA was added to a 10% FBS-DMEM (H-Glc) to prepare a sample medium. Using an aspirator, the medium of HepG2 cells prepared in the same manner as in [Preparation of HeG2 cells] in Experimental Example 1 and cultured in a 96-well microplate was removed. Each well was washed twice with 100 $\mu$l/well of a serum-free DMEM (H-Glc). Then, a sample medium containing siRNA was added at 100 $\mu$l/well. In this case, siRNA was added at 1 pmol/well. This was cultured under conditions of 37°C and 5% $CO_2$. Here, the sample medium containing siRNA was added 72 hours after HepG2 cells were seeded in the 96-well microplate.

The degree of growth of cells to which each sample was added was tested by the same method as in the [Growth ability test] in Experimental Example 1.

[Results]

The above results are shown in Table 2 and Fig. 5. Here, #1, #2, and #3 in Table 2 and Fig. 5 indicate siRNAs that target different RNA sequences of mRNA of the same gene. As can be clearly understood from the results of Table 2 and Fig. 5, comparing the control group in which no siRNA was added, the growth of HepG2 cells was inhibited in the siRNA-added group. Particularly, even when a reproducibility experiment was performed, VDAC1#3 and SP1#3 exhibiting a significant growth ability inhibition effect were used for verification in Experimental Example 3 and onwards.

[Table 2]

|  | % with respect to control group | standard error |
|---|---|---|
| control group | 100.0 | 10.6 |
| VDAC1 #1 | 65.3 | 17.6 |
| VDAC1 #2 | 81.8 | 8.0 |
| VDAC1 #3 | 37.8 | 8.5 |
| IGF2BP1 #1 | 40.9 | 6.5 |
| IGF2BP1 #2 | 85.6 | 8.8 |
| IGF2BP1 #3 | 77.2 | 9.4 |
| SP1 #1 | 85.1 | 12.9 |
| SP1 #2 | 67.8 | 7.7 |
| SP1 #3 | 35.9 | 1.3 |
| random #1 | 101.9 | 12.2 |
| random #2 | 66.0 | 12.1 |
| DOX liposome | 8.4 | 2.1 |

<Experimental Example 3; examination of cell growth inhibitory effect of peptide-binding to siRNA-encapsulated liposome>

In this experimental example, when siRNA was encapsulated into a liposome and administered to HepG2 cells, the growth inhibitory effect was checked. In this experimental example, SP1#3 was used as siRNA to be encapsulated.

[Preparation of liposome]

As lyophilized liposomes, Lipocapsulater FD-U PL (dioleyl phosphatidylcholine:cholesterol=70:30, commercially available from Katayama Chemical Industries), and Lipocapsulater FD-S PL(distearoyl phosphatidylcholine:cholesterol=70:30, commercially available from Katayama Chemical Industries) were used. A solution was prepared so that the lyophilized liposome was at a concentration of 1 mg/40 $\mu$l in PBS(-). The siRNA solution was added thereto and mixed with a liposome solution. In addition, an RNA encapsulating solution (liposome encapsulation kit, Lipocapsulater, commercially available from Hygieia Bioscience) 1.5 times as much as this mixed solution was mixed. After mixing, the mixture was left at room temperature for 30 minutes. In order to remove unencapsulated siRNA, the siRNA-encapsulated liposome solution was transferred to a centrifugal concentration tube VIVASPIN500 (commercially available from Funakoshi), and centrifuged under conditions of 13,000 G×10 min, 25°C.

[Preparation of siRNA-encapsulated peptide-binding liposome]

A solution of a peptide having an amino acid sequence shown in SEQ ID NO: 1 was added to a 20 mM borate buffer solution (pH 8.6). In order to add a peptide having an amino acid sequence shown in SEQ ID NO: 1 to a liposome, the following two adding processes were performed. Here, the powder DSPE-PEG2000-NHS (1,2-distearoyl-sn-glycero-3-phosphatidylethanolamine-N-[carboxy(polyethylene glycol) 2000, NHS ester], 880138, Croda International Plc.) was used by being dissolved in PBS.

(Adding process 1: adding complex lipid to liposome before reaction with peptide)

In order to add DSPE-PEG2000-NHS to a lipid membrane of a liposome, a DSPE-PEG2000-NHS solution was added to the siRNA-encapsulated liposome solution. A mixed solution containing siRNA-encapsulated Lipocapsulater FD-U PL and DSPE-PEG2000-NHS was heated at 37°C for 30 minutes. A mixed solution containing siRNA-encapsulated Lipocapsulater FD-S PL and DSPE-PEG2000-NHS was heated at 60°C for 30 minutes. In the siRNA-encapsulated Lipocapsulater FD-S PL, since the phase transition temperature of the lipid was relatively high, the temperature was raised to 60°C. A solution of a peptide having an amino acid sequence shown in SEQ ID NO: 1, SEQ ID NO: 2, or SEQ ID NO: 5 was added to this mixed solution, the mixture was left at room temperature for 2 hours, and the NHS group and the amino group of the peptide were reacted.

(Adding process 2: adding complex lipid to liposome after reaction with peptide)

A solution of a peptide having an amino acid sequence shown in SEQ ID NO: 1, SEQ ID NO: 2, or SEQ ID NO: 5 and a DSPE-PEG2000-NHS solution were mixed, the mixture was left at room temperature for 2 hours, and thus the NHS group and the amino group of the peptide were reacted. In addition, a DSPE-PEG2000-peptide solution was mixed with the siRNA-encapsulated liposome solution. A mixed solution containing siRNA-encapsulated Lipocapsulater FD-U PL and DSPE-PEG2000-peptide was heated at 37°C for 30 minutes. A mixed solution containing siRNA-encapsulated Lipocapsulater FD-S PL and DSPE-PEG2000-peptide was heated at 60°C for 30 minutes.

[Addition of HeG2 cells to peptide-binding liposome]

The prepared peptide-binding siRNA-encapsulated liposome was mixed with a DMEM (H-Glc) medium. Using the exosome-depleted FBS, the final concentration was adjusted to 1% for FBS (exosome-depleted) DMEM (G-Glc). HepG2 cells prepared in the same manner as in [Preparation of HeG2 cells] in Experimental Example 1 were added so that 100 μl of the peptide-binding liposome solution per well for each group n=8. Here, the siRNA-encapsulated liposome solution was added 72 hours after HepG2 cells were seeded in the 96-well microplate.
The degree of growth of cells to which each sample was added was tested by the same method as in the [Growth ability test] in Experimental Example 1.

[Results]

As shown in Table 3A and Fig. 6A, when siRNA was encapsulated into a liposome derived from Lipocapsulater FD-U PL and a peptide was added, inhibition of HeG2 cell growth was enhanced. Such a growth inhibitory ability was observed at the same level in both the adding process 1 and the adding process 2. In addition, as shown in Table 4A and Fig. 6D, when siRNA was encapsulated into a liposome of Lipocapsulater FD-S PL and a peptide was added, inhibition of HeG2 cell growth was enhanced. In this case, it was suggested that the sample prepared in the process 1 had a stronger growth inhibition function. In Lipocapsulater FD-S PL, 70% of lipids constituting liposomes were saturated lipid distearoyl phosphatidylcholine (DSPE), and the fluidity of the lipid bilayer was low. In the process 2, an attempt was made to bind DSPE-PEG2000-NHS and a peptide and then introduce it into a liposome. However, the bulky DSPE-PEG2000-peptide may not enter a membrane with low fluidity. As a result, it was considered that the rate of peptides addition to liposomes decreased, and a cell growth inhibition function may also become weak. In addition, as shown in Table 3B, Table 3C, Fig. 6B, and Fig. 6C, and Table 4B, Table 4C, Fig. 6E, and Fig. 6F, when peptides having amino acid sequences shown in SEQ ID NO: 2 and SEQ ID NO: 5 were added, inhibition of HeG2 cell growth was enhanced in the results as in the case of adding a peptide having an amino acid sequence shown in SEQ ID NO: 1.

EP 4 393 957 A1

[Table 3A]

| | % with respect to control group | standard error |
|---|---|---|
| control group | 100.0 | 6.6 |
| liposome (DOPC:cholesterol = 70:30) | | |
| no additive | 65.2 | 8.9 |
| siRNA (SP1 # 3) 6.67 $\mu$ g/group | 41.6 | 6.8 |
| siRNA (SP1 # 3) 6.67 $\mu$ g/group (adding process 1) | 30.4 | 2.9 |
| siRNA (SP1 # 3) 6.67 $\mu$ g/group (adding process 2) | 30.0 | 5.6 |

[Table 3B]

| | % with respect to control group | standard error |
|---|---|---|
| control group | 100.0 | 6.5 |
| liposome (DOPC:cholesterol = 70:30) | | |
| no additive | 66.3 | 9.4 |
| siRNA (SP1 #3) 6.67 $\mu$ g | 43.3 | 6.7 |
| siRNA (SP 1 #3) 6.67 $\mu$ g (adding process 1; SEQ ID No. 2) | 32.9 | 2.5 |
| siRNA (SP1 #3) 6.67 $\mu$ g (adding process 2 ; SEQ ID No. 2) | 37.2 | 4.0 |

[Table 3C]

| | % with respect to control group | standard error |
|---|---|---|
| control group | 100.0 | 8.6 |
| liposome (DOPC:cholesterol = 70:30) | | |
| no additive | 80.7 | 8.5 |
| siRNA (SP1 #3) 6.67 $\mu$ g | 54.5 | 5.0 |
| siRNA (SP1 #3) 6.67 $\mu$ g (adding process 1; SEQ ID No. 5) | 43.2 | 3.0 |
| siRNA (SP1 #3) 6.67 $\mu$ g (adding process 2; SEQ ID No. 5) | 41.9 | 6.8 |

[Table 4A]

| | % with respect to control group | standard error |
|---|---|---|
| control group | 100.0 | 14.4 |
| liposome (DSPC:cholesterol = 70:30) | | |
| no additive | 97.3 | 7.6 |
| siRNA (SP1 #3) 6.67 $\mu$ g/group | 88.4 | 15.6 |
| siRNA (SP1 #3) 6.67 $\mu$ g/group (adding process 1) | 49.5 | 7.1 |
| siRNA (SP1 # 3) 6.67 $\mu$ g/group (adding process 2) | 11.2 | 11.2 |

22

[Table 4B]

| | % with respect to control group | standard error |
|---|---|---|
| control group | 100.0 | 14.8 |
| liposome (DSPC:cholesterol = 70:30) | | |
| no additive | 97.3 | 3.0 |
| siRNA (SP1 #3) 6.67 $\mu$ g/group | 88.7 | 15.6 |
| siRNA (SP1 #3) 6.67 $\mu$ g/group (adding process 1: SEQ ID No. 2) | 48.4 | 7.1 |
| siRNA (SP1 #3) 6.67 $\mu$ g/group (adding process 2: SEQ ID No. 2) | 68.8 | 11.0 |

[Table 4C]

| | % with respect to control group | standard error |
|---|---|---|
| control group | 100.0 | 16.3 |
| liposome (DSPC:cholesterol = 70:30) | | |
| no additive | 94.1 | 3.5 |
| siRNA (SP1 #3) 6.67 $\mu$ g/ group | 88.1 | 14.5 |
| siRNA (SP1 #3) 6.67 $\mu$ g/ group (adding process 1: SEQ ID No. 5) | 51.7 | 7.2 |
| siRNA (SP1 #3) 6.67 $\mu$ g/ group (adding process 2: SEQ ID No. 5) | 62.3 | 7.8 |

<Experimental Example 4; examination of influence of MSC-derived exosome on cell growth>

In order to examine the influence of exosomes on growth of HepG2 cells, the influence of HepG2 on cell growth was verified using commercially available exosomes. Here, the results indicated that human mesenchymal stem cells (MSC) influenced growth of other cells and exosomes were suggested to be responsible for some of effects. Therefore, MSC-derived exosomes were incorporated into HepG2 cells, and examined their influences on cell growth.

[Preparation and addition of exosome]

Purchased bone marrow (BM)-derived MSC-derived exosomes (PCS-500-012, SBI, American Type Culture Collection (ATCC)) were suspended in an assay medium (final concentration of 1% for FBS (exosome-depleted) DMEM (H-Glc)). Using an aspirator, the medium of HepG2 cells prepared in the same manner as in [Preparation of HeG2 cells] in Experimental Example 1 and cultured in a 96-well microplate was removed. Each well was washed twice with 100 $\mu$l/well of a serum-free DMEM (H-Glc). The MSC-derived exosome solution was added at 50 $\mu$l/well for each group n=6. Here, the MSC-derived exosome solution was added 72 hours after HepG2 cells were seeded in the 96-well microplate.

[Results]

As shown in Table 5 and Fig. 7, compared to the control group in which no liposomes or exosomes were added, cell growth was significantly inhibited in the group in which 40 $\mu$g/ml of an MSC-derived exosome solution was added. Since exosomes contained molecules having a physiological function such as proteins and miRNA, it was thought that these functions inhibited growth of HepG2 cells.

[Table 5]

|  | % with respect to control group | standard error |
|---|---|---|
| control group | 100.0 | 6.8 |
| BM-MSC-derived exosome 40 $\mu$ g/ml | 42.5 | 3.2 |
| control group liposome 40 $\mu$ g | 67.6 | 8.3 |
| DOX liposome 40 $\mu$ g | 13.9 | 1.3 |

<Experimental Example 5; examination of influence of peptide-binding MSC-derived exosome solution on cell growth>

In Experimental Example 4, it was confirmed that MSC-derived exosomes inhibited growth of HepG2 cells. It was expected that, when molecules having a growth inhibition function contained in the MSC-derived exosomes were efficiently delivered into the cytoplasm of HepG2 cells using a predetermined peptide, the function of the MSC-derived exosome was further enhanced. Therefore, a peptide having an amino acid sequence shown in SEQ ID NO: 1 was added to MSC-derived exosomes, and the influence on growth of HepG2 cells was examined.

In addition, the same method was applied to exosomes derived from HEK293 cells, which are derived from kidney cells, a peptide having an amino acid sequence shown in SEQ ID NO: 1 was added to the HKE293 cell-derived exosomes, and the influence on growth of HepG2 cells was examined.

[Preparation and addition of peptide-binding MSC-derived exosome solution]

A solution of a peptide having an amino acid sequence shown in SEQ ID NO: 1 was added to a 20 mM borate buffer solution (pH 8.6). In addition, Lipofectamine (registered trademark) (solution for transfection) was dissolved in a serum-free DMEM to prepare a 10% Lipofectamine solution. An exosome solution (SCRC-4000-EXM, American Type Culture Collection (ATCC)) containing exosomes secreted from adipose-derived stem cells Adipo MSC, the peptide solution, and a 10% Lipofectamine solution were mixed. After mixing with a vortex mixer, the mixture was left at room temperature for 20 minutes. A serum-free DMEM and a 10% FBS (exosome-depleted) DMEM (H-Glc) were added, and the concentration of the medium was adjusted so that the final concentration was 1% for the FBS (exosome-depleted) DMEM (G-Glc). Using an aspirator, the medium of HepG2 cells prepared in the same manner as in [Preparation of HeG2 cells] in Experimental Example 1 was removed. Each well was washed twice with 100 $\mu$l/well of a serum-free DMEM (H-Glc). The peptide-binding MSC-derived exosome solution was added at 50 $\mu$l/well for each group n=6. Here, the peptide-binding MSC-derived exosome solution was added 24 hours after HepG2 cells were seeded in the 96-well microplate.

[Preparation and addition of peptide-binding HEK293 cell-derived exosome solution]

The same experiment was performed according to the method described in [Preparation and addition of peptide-binding MSC-derived exosome solution] using HEK293 cells derived from human kidney cells instead of the adipose-derived stem cells Adipo MSC. More specifically, $5.0 \times 10^5$ HEK293 cells were seeded in a 100 mm culture dish. The medium for cell culture was prepared using an HE100 medium (GMEP) containing a glutamine, penicillin, and streptomycin solution, with a total amount of 10 ml. 3 days after culture started, the medium was removed by suction, and 10 ml of the new HE100 medium was added. The supernatant was collected 7 days after seeding. Exosomes were collected according to the protocol of Capturem Exosome Isolation Kit (Cell Culture) (commercially available from Takara Bio Inc.).

[Results]

The results of the experiment using adipose-derived stem cells Adipo MSC are shown in Table 6A and Fig. 8A, and the results of the experiment using HEK293 cells are shown in Table 6B and Fig. 8B. As shown in Table 6A and Fig. 8A, in the group in which 40 $\mu$g/ml of Adipo MSC-derived exosomes were added, HepG2 cell growth was promoted unlike the results for BM MSC-derived exosomes in Experimental Example 4. This was inferred to be due to differences in the functions of exosomes depending on differences in properties of cells from which they were derived. That is, even if MSC cells were the same, there were differences in properties of cells due to their different origins. Therefore, it was thought that the types and structures of proteins and nucleic acids contained in exosomes changed, and the influence on growth of HepG2 cells changed. Here, similar results were also found in Table 6B and Fig. 8B, and it was suggested that exosomes derived from HEK293 cells also had similar functions.

[Table 6A]

|  | % with respect to control group | standard error |
|---|---|---|
| control group | 100.0 | 7.6 |
| peptide | 147.2 | 8.8 |
| Lipofectamine 2% | 117.7 | 10.9 |
| Adipo-MSC-derived exosome 40 $\mu$ g/m l | 281.4 | 22.5 |
| Adipo-MSC-derived exosome 40 $\mu$ g/m l + peptide + Lipofectamine 2% | 40.1 | 4.5 |
| DOX liposome 40 $\mu$M | 61.7 | 5.5 |

[Table 6B]

|  | % with respect to control group | standard error |
|---|---|---|
| control group | 100.0 | 7.1 |
| peptide | 141.3 | 8.2 |
| Lipofectamine 2% | 112.6 | 9.1 |
| exosome 40 $\mu$ g/ml | 161.5 | 13.1 |
| exosome 40 $\mu$ g/ml + peptide (SEQ ID No. 1) | 151.6 | 18.6 |
| DOX liposome 40 $\mu$ M | 59.9 | 4.4 |

However, the function of the growth promoting ability of such Adipo MSC-derived exosomes was reversed by adding a peptide having an amino acid sequence shown in SEQ ID NO: 1 to exosomes. That is, in the Adipo MSC-derived exosome group in which the peptide was not added, HepG2 cell growth was promoted, but growth of HepG2 cells was inhibited in the Adipo MSC-derived exosomes to which the peptide was added.

<Experimental Example 6; examination of influence of peptide-binding MSC-derived exosome solution on cell growth>

In order to verify reproducibility of the reversal phenomenon of the cell growth promoting ability in Experimental Example 5, a similar examination was performed using Adipo MSC-derived exosomes (in addition to those obtained by adding a peptide having an amino acid sequence shown in SEQ ID NO: 1, those obtained by adding peptides having amino acid sequences shown in SEQ ID NO: 2 to SEQ ID NO: 5 were added to examination targets) and BM MSC-derived exosomes in Experimental Example 4. Regarding culture conditions for cells to which exosomes were delivered, in addition to following the method described in Experimental Example 1, the number of Hela cells seeded and the number of Caco2 cells seeded were adjusted to 5,000 cells/100 $\mu$l/well (96-well microplate) and 5,000 cells/100 $\mu$l/well (96-well microplate), respectively. The results are shown in Table 7A to Table 7E and Fig. 9A to Fig. 9E. When BM MSC-derived exosomes were added, growth of HepG2 cells was inhibited, and when Adipo MSC-derived exosomes were added, growth of HepG cells was promoted. In addition, when peptides having amino acid sequences shown in SEQ ID NOs: 1, 2, and 5 were added to the exosomes, the influence of respective exosomes on growth of HepG2 cells was almost reversed. Therefore, a similar function reversal phenomenon was observed not only in the Adipo MSC-derived exosomes but also in the BM MSC-derived exosomes due to the addition of the peptides.

[Table 7A]

|  | % with respect to control group | standard error |
|---|---|---|
| control group | 100.0 | 9.6 |
| Adipo MSC-derived exosome 30 $\mu$ g/m l | 132.1 | 6.9 |

(continued)

|  | % with respect to control group | standard error |
|---|---|---|
| Adipo MSC-derived exosome 30 $\mu$ g/m l + peptide + Lipofectamine 2% | 61.4 | 12.8 |
| BM-MSC-derived exosome 30 $\mu$ g/m l | 76.5 | 1.9 |
| BM-MSC-derived exosome 30 $\mu$ g/m l + peptide + Lipofectamine 2% | 142.8 | 6.1 |
| DOX liposome 40 $\mu$ M | 35.2 | 6.7 |

[Table 7B]

|  | % with respect to control group | standard error |
|---|---|---|
| control group | 100.0 | 9.4 |
| Adipo MSC-derived exosome 30 $\mu$ g/ml | 127.7 | 6.8 |
| Adipo MSC-derived exosome 30 $\mu$ g/ml + peptide (SEQ No. 2) | 59.0 | 12.1 |
| BM MSC-derived exosome 30 $\mu$ g/ml | 77.4 | 3.2 |
| BM MSC-derived exosome 30 $\mu$ g/ml + peptide (SEQ No. 2) | 139.4 | 6.5 |
| DOX liposome 40 $\mu$ M | 32.3 | 7.3 |

[Table 7C]

|  | % with respect to control group | standard error |
|---|---|---|
| control group | 100.0 | 11.1 |
| Adipo MSC-derived exosome 30 $\mu$ g/ml | 137.5 | 7.5 |
| Adipo MSC-derived exosome 30 $\mu$ g/ml + peptide (SEQ No. 3) | 49.8 | 4.6 |
| BM MSC-derived exosome 30 $\mu$ g/ml | 80.9 | 2.5 |
| BM MSC-derived exosome 30 $\mu$ g/ml + peptide (SEQ No. 3) | 150.2 | 7.3 |
| DOX liposome 40 $\mu$ M | 29.8 | 5.3 |

[Table 7D]

|  | % with respect to control group | standard error |
|---|---|---|
| control group | 100.0 | 9.7 |
| Adipo MSC-derived exosome 30 $\mu$ g/ml | 129.2 | 8.4 |
| Adipo MSC-derived exosome 30 $\mu$ g/ml + peptide (SEQ No. 5) | 74.1 | 4.2 |
| BM MSC-derived exosome 30 $\mu$ g/ml | 78.5 | 2.2 |
| BM MSC-derived exosome 30 $\mu$ g/ml + peptide (SEQ No. 5) | 95.1 | 3.8 |
| DOX liposome 40 $\mu$ M | 17.0 | 3.8 |

[Table 7E]

|  | % with respect to control group | standard error |
|---|---|---|
| control group | 100.0 | 9.4 |

(continued)

| | % with respect to control group | standard error |
|---|---|---|
| Adipo MSC-derived exosome 30 $\mu$ g/ml | 136.1 | 10.7 |
| Adipo MSC-derived exosome 30 $\mu$ g/ml + peptide (SEQ No. 4) | 59.0 | 5.6 |
| BM MSC-derived exosome 30 $\mu$ g/ml | 91.7 | 5.7 |
| BM MSC-derived exosome 30 $\mu$ g/ml + peptide (SEQ No. 4) | 112.0 | 8.2 |
| DOX liposome 40 $\mu$ M | 15.5 | 3.9 |

<Experimental Example 7; verification of membrane fusion using fluorescence label>

In order to analyze each peptide-binding hybrid liposome exosome particle, flow cytometry was performed using a fluorescence labeling reagent that causes a fluorescence resonance energy transfer (FRET) phenomenon. In this experimental example, an anti-CD63 antibody PECy7, which is an antibody for detecting CD63 as a marker molecule for exosomes, was used. In addition, exosomes were collected from BM MSC, and used to verify membrane fusion.

[Preparation of liposome]

Using a method according to adding process 2 in [Preparation of siRNA-encapsulated peptide-binding liposome] in Experimental Example 3, except that siRNA was not encapsulated, peptide-binding liposomes were prepared using a FAM-labeled peptide having an amino acid sequence shown in SEQ ID NO: 3, an Antennapedia FITC-labeled peptide having an amino acid sequence shown in SEQ ID NO: 5, Lipocapsulater FD-U PL, and Lipocapsulater FD-S PL.

[Preparation of BM MSC-derived exosome]

$1.2\times10^5$ BM MSC cells were seeded in a T75 flask. The medium was prepared using MesenCult ACF Plus Medium Kit (commercially available from Veritas Corporation), with a total amount of 15 ml. After 3 days, the medium was removed by suction, and 15 ml of a new MesenCult ACF Plus Medium was added. 7 days after seeding, the supernatant was collected. Exosomes were collected according to the protocol of Capturem Exosome Isolation Kit (Cell Culture) (commercially available from Takara Bio Inc.).

[Preparation of peptide-binding hybrid liposome exosome]

The peptide-added liposome and exosome solutions were mixed, and a PEG 8000 solution was added so that the concentration was 30%. The mixture was left at 40°C for 2 hours, and the peptide-binding liposome and the exosome were fused. In the control group, a PBS buffer solution was added instead of the PEG 8000 solution.

[Detection of peptide-binding hybrid liposome exosome by flow cytometry]

A sample for flow cytometry was prepared according to the protocol of PS Capture exosome flow cytometry kit (commercially available from FUJIFILM Wako Pure Chemical Corporation). In addition, for flow cytometry, Attune (registered trademark) Acoustic Focusing Flow Cytometer (commercially available from Thermo Fisher Scientific Inc.) was used. As antibodies for exosome detection, PE-Cy7-labeled anti-human CD63 mouse antibody clone H5C6 RUO (commercially available from Novus Biologicals) was used.

[Results]

Fig. 10 shows diagrams of the results of flow cytometry of hybrid liposome exosomes of Lipocapsulater FD-U PL to which a peptide shown in SEQ ID NO: 3 was added and an exosome. Fig. 11 shows diagrams of the results of flow cytometry of hybrid liposome exosomes of Lipocapsulater FD-U PL to which a peptide shown in SEQ ID NO: 5 was added and an exosome. Fig. 12 shows diagrams of the results of flow cytometry of hybrid liposome exosomes of Lipocapsulater FD-S PL to which a peptide shown in SEQ ID NO: 5 was added and an exosome. Taking Fig. 10 as examples, (D) in Fig. 10 shows the results of flow cytometry performed on peptide-binding hybrid liposome exosomes, and as control groups, (A) shows the results of flow cytometry performed when no peptide was bound to a liposome and an operation of fusing a liposome with an exosome was not performed, (B) shows the results of flow cytometry performed when a peptide was bound to a liposome and an operation of fusing a liposome with an exosome was not performed, and (C) shows the results of flow cytometry performed when no peptide was bound to a liposome and an operation of fusing a liposome with an exosome was performed. Among these, (a) shows the relationship between the intensity (horizontal axis) of the exosome-labeled fluorescent dye and the count number (vertical axis), (b) shows the relationship between the intensity (horizontal axis) of the peptide-labeled fluorescent dye and the count number (vertical axis), and (c) shows scatter plots of the intensity (horizontal axis) of the peptide-labeled fluorescent dye and the intensity (vertical axis) of the exosome-labeled fluorescent dye. As can be clearly understood by comparing the percentage (6.551%) of fractions in which both the intensity (horizontal axis) of the peptide-labeled fluorescent dye and the intensity (vertical axis) of the exosome-labeled fluorescent dye were high in (c) in (D) with (A),(B) and (C) (0.0638%, 0.0696% and 1.403%, respectively), in (D), the percentage of vesicles in which both the intensity (horizontal axis) of the peptide-labeled fluorescent dye and the intensity (vertical axis) of the exosome-labeled fluorescent dye, which were derived from the peptide-binding hybrid liposome exosome, were high was larger.

Here, the above experiment was performed in the same manner using rabbit polyclonal antibodies that specifically bind to the above peptide as primary antibodies against the above peptide, and fluorescently labeling peptides with fluorescence-labeled anti-rabbit antibodies. However, even in such cases, experiment results similar to the above experiment results were obtained. The results are shown in Fig. 15 and Fig. 16. In this case, the rabbit polyclonal antibodies that specifically bind to the above peptide were prepared according to the method described in the above (Method of producing antibodies against exosome marker proteins and peptides constituting peptide-binding lipid derivatives). Although the present invention is not bound by any theory, it is speculated that, since polyclonal antibodies are a mixture of antibodies that recognize a plurality of sites on a peptide as epitopes, if polyclonal antibodies are prepared according to a general operation, test results equivalent to the above test results are obtained. Here, regarding explanations of reference numerals in the drawings, explanations of reference numerals in Fig. 10 will be applied as examples.

<Experimental Example 8; examination of cell growth inhibitory ability of peptide-binding hybrid liposome exosome>

Adipo MSC-derived exosomes were hybridized with (peptide-binding) siRNA-encapsulated liposomes. The prepared (peptide-binding) hybrid liposome exosomes were added to HepG2 cells, Hela cells, and Caco2 cells, and the influence on growth was examined. Here, peptides were added to liposomes rather than to Adipo MSC-derived exosomes. Regarding culture conditions for cells to which exosomes were delivered, in addition to following the method described in Experimental Example 1, the number of Hela cells seeded and the number of Caco2 cells seeded were adjusted to 5,000 cells/100 μl/well (96-well microplate) and 5,000 cells/100 μl/well (96-well microplate), respectively.

[Preparation of peptide-binding hybrid liposome exosome]

Using a method according to adding process 2 in [Preparation of siRNA-encapsulated peptide-binding liposome] in Experimental Example 3, except that siRNA was not encapsulated, peptide-binding liposomes were prepared using peptides having amino acid sequences shown in SEQ ID NO: 1, SEQ ID NO: 2, SEQ ID NO: 3 and SEQ ID NO: 5, Lipocapsulater FD-U PL, and Lipocapsulater FD-S PL.

The liposome to which the peptide was added and an Adipo MSC-derived exosome solution were mixed. A PEG 8000 solution was added so that the concentration was 30% and left at 40°C for 2 hours, and peptide-added liposomes and Adipo MSC-derived exosomes were fused. In the control group, a PBS buffer solution was added instead of the PEG 8000 solution. A serum-free DMEM and a 10% FBS (exosome-depleted) DMEM (H-Glc) were added, and the concentration of the medium was adjusted so that the final concentration was 1% for the FBS (exosome-depleted) DMEM (G-Glc). Using an aspirator, the medium of HepG2 cells prepared in the same manner as in [Preparation of HeG2 cells] in Experimental Example 1 was removed. Each well was washed twice with 100 μl/well of a serum-free DMEM (H-Glc). The peptide-binding MSC-derived exosome solution was added at 50 μl/well for each group n=6. Here, the peptide-binding MSC-derived exosome solution was added 48 hours after HepG2 cells were seeded in the 96-well microplate.

The degree of growth of cells to which each sample was added was tested by the same method as in the [Growth ability test] in Experimental Example 1.

[Results]

As shown in Table 8A to Table 8E, and Fig. 13A to Fig. 13E, it was confirmed that the peptide-binding hybrid liposome exosomes exhibited a statistically significantly enhanced growth inhibitory effect compared to exosomes not fused with liposomes and hybrid liposome exosomes to which no peptide was added. The results indicate that a desired effect was enhanced by encapsulating an active component in a peptide-added liposome and fusing it with an exosome.

Here, the results of the test executed by performing the same operation by the method according to the adding process 1 in [Preparation of siRNA-encapsulated peptide-binding liposome] in Experimental Example 3 in [Preparation of peptide-binding hybrid liposome exosome] are shown in Table 9A to Table 9E, and Fig. 14A to Fig. 14E.

[Table 8A]

|  | % with respect to control group | standard error |
|---|---|---|
| control group | 100.0 | 2.4 |
| Adipo-MSC-derived exosome SP1 siRNA-encapsulated liposome no fusion | 91.2 | 2.0 |
| Adipo-MSC-derived exosome SP1 siRNA-encapsulated liposome fusion | 86.1 | 2.2 |
| Adipo-MSC-derived exosome SP1 siRNA-encapsulated liposome fusion peptide no adding | 86.0 | 2.5 |
| Adipo-MSC-derived exosome SP1 siRNA-encapsulated liposome fusion peptide adding | 70.2 | 6.1 |

[Table 8B]

|  | % with respect to control group | standard error |
|---|---|---|
| control group | 100.0 | 2.4 |
| Adipo-MSC-derived exosome SP1 siRNA-encapsulated liposome no fusion | 89.1 | 4.9 |
| Adipo-derived exosome SP1 siRNA-encapsulated liposome fusion | 87.1 | 3.6 |
| Adipo-MSC-derived exosome SP1 siRNA-encapsulated liposome fusion peptide no adding | 79.1 | 4.8 |
| Adipo-MSC-derived exosome SP1 siRNA-encapsulated liposome fusion peptide adding (SEQ No. 2) | 62.1 | 9.0 |

[Table 8C]

|  | % with respect to control group | standard error |
|---|---|---|
| control group | 100.0 | 3.4 |
| Adipo-MSC-derived exosome SP1 siRNA-encapsulated liposome no fusion | 88.6 | 3.8 |
| Adipo-derived exosome SP1 siRNA-encapsulated liposome fusion | 89.8 | 2.1 |
| Adipo-MSC-derived exosome SP1 siRNA-encapsulated liposome fusion peptide no adding | 89.2 | 5.4 |
| Adipo-MSC-derived exosome SP1 siRNA-encapsulated liposome fusion peptide adding (SEQ No. 3) | 60.9 | 8.1 |

[Table 8D]

| | % with respect to control group | standard error |
|---|---|---|
| control group | 100.0 | 4.2 |
| Adipo-MSC-derived exosome SP1 siRNA-encapsulated liposome no fusion | 94.5 | 4.1 |
| Adipo-derived exosome SP1 siRNA-encapsulated liposome fusion | 81.7 | 1.8 |
| Adipo-MSC-derived exosome SP1 siRNA-encapsulated liposome fusion peptide no adding | 92.1 | 4.8 |
| Adipo-MSC-derived exosome SP1 siRNA-encapsulated liposome fusion peptide adding (SEQ No. 5) | 58.5 | 8.2 |

[Table 8E]

| | % with respect to control group | standard error |
|---|---|---|
| control group | 100.0 | 1.2 |
| Adipo-MSC-derived exosome SP1 siRNA-encapsulated liposome no fusion | 91.7 | 2.7 |
| Adipo-derived exosome SP1 siRNA-encapsulated liposome fusion | 81.3 | 1.3 |
| Adipo-MSC-derived exosome SP1 siRNA-encapsulated liposome fusion peptide no adding | 83.9 | 9.1 |
| Adipo-MSC-derived exosome SP1 siRNA-encapsulated liposome fusion peptide adding (SEQ No. 4) | 64.2 | 8.2 |

[Table 9A]

| | % with respect to control group | standard error |
|---|---|---|
| control group | 100.0 | 3.9 |
| exosome SP1 siRNA-encapsulated liposome no fusion | 98.8 | 3.6 |
| exosome SP1 siRNA-encapsulated liposome fusion | 85.1 | 6.9 |

(continued)

|  | % with respect to control group | standard error |
|---|---|---|
| exosome<br>SP1 siRNA-encapsulated liposome fusion peptide no adding | 85.7 | 2.9 |
| exosome<br>SP1 siRNA-encapsulated liposome fusion peptide adding | 76.0 | 4.4 |

[Table 9B]

|  | % with respect to control group | standard error |
|---|---|---|
| control group | 100.0 | 3.5 |
| exosome<br>SP1 siRNA-encapsulated liposome no fusion | 95.9 | 6.8 |
| exosome<br>SP1 siRNA-encapsulated liposome fusion | 85.7 | 5.4 |
| exosome<br>SP1 siRNA-encapsulated liposome fusion peptide no adding | 86.4 | 4.4 |
| exosome<br>SP1 siRNA-encapsulated liposome fusion peptide adding (SEQ No. 2) | 76.2 | 5.8 |

[Table 9C]

|  | % with respect to control group | standard error |
|---|---|---|
| control group | 100.0 | 4.1 |
| exosome<br>SP1 siRNA-encapsulated liposome no fusion | 95.6 | 3.4 |
| exosome<br>SP1 siRNA-encapsulated liposome fusion | 80.7 | 7.4 |
| exosome<br>SP1 siRNA-encapsulated liposome fusion peptide no adding | 83.7 | 4.6 |
| exosome<br>SP1 siRNA-encapsulated liposome fusion peptide adding (SEQ No. 3) | 73.9 | 4.2 |

[Table 9D]

| | % with respect to control group | standard error |
|---|---|---|
| control group | 100.0 | 5.0 |
| exosome SP1 siRNA-encapsulated liposome no fusion | 95.1 | 3.7 |
| exosome SP1 siRNA-encapsulated liposome fusion | 83.7 | 6.8 |
| exosome SP1 siRNA-encapsulated liposome fusion peptide no adding | 82.0 | 1.3 |
| exosome SP1 siRNA-encapsulated liposome fusion peptide adding (SEQ No. 5) | 81.7 | 5.5 |

[Table 9E]

| | % with respect to control group | standard error |
|---|---|---|
| control group | 100.0 | 3.7 |
| exosome SP1 siRNA-encapsulated liposome no fusion | 90.6 | 5.4 |
| exosome SP1 siRNA-encapsulated liposome fusion | 82.0 | 6.7 |
| exosome SP1 siRNA-encapsulated liposome fusion peptide no adding | 83.4 | 4.3 |
| exosome SP1 siRNA-encapsulated liposome fusion peptide adding (SEQ No. 4) | 74.5 | 4.4 |

**Claims**

1. A peptide-binding hybrid liposome exosome, comprising in a lipid bilayer:

   an exosome marker protein selected from CD63, CD81, and CD9, and
   peptide-binding lipid derivatives in which a peptide binds to a hydrophilic part of a complex lipid.

2. The peptide-binding hybrid liposome exosome according to claim 1,

   wherein the peptide has 10 to 30 amino acid residues,
   wherein the peptide comprises a repeating sequence composed of 3 to 4 amino acid residues comprising at least one hydrophobic amino acid residue selected from an alanine residue, a leucine residue, and a methionine residue and at least one amino acid residue selected from a glutamic acid residue, an aspartic acid residue, a glutamine residue, and an asparagine residue, and

wherein at least one of amino acid residues constituting the repeating sequence is one of a glutamic acid residue, an aspartic acid residue, a glutamine residue, and an asparagine residue.

3. The peptide-binding hybrid liposome exosome according to claim 1,

   wherein the peptide has 10 to 30 amino acid residues, and
   wherein the peptide has at least one basic amino acid residue.

4. The peptide-binding hybrid liposome exosome according to claim 3,
   wherein the peptide has 2 to 5 consecutive basic amino acid residues.

5. The peptide-binding hybrid liposome exosome according to claim 3 or 4,
   wherein the peptide does not contain an acidic amino acid residue.

6. The peptide-binding hybrid liposome exosome according to claim 1 or 2,
   wherein the peptide is any of peptides having amino acid sequences shown in SEQ ID NOs: 1 to 5.

7. The peptide-binding hybrid liposome exosome according to claim 1 or 2,

   wherein the complex lipid is a complex lipid having, at an end, any reactive group selected from the group consisting of primary amino groups, carboxyl groups, hydroxy groups, phosphate groups, and thiol groups, and
   wherein the complex lipid is at least one selected from the group consisting of sphingophospholipids, glycosphingolipids, glycerophospholipids, glyceroglycolipids, ether type phospholipids, and ether type glycolipids.

8. The peptide-binding hybrid liposome exosome according to claim 1 or 2,
   wherein the peptide and the lipid derivatives are bonded by an NHS ester crosslinking reaction, carbodiimide crosslinking reaction, or imidoester crosslinking reaction method.

9. The peptide-binding hybrid liposome exosome according to claim 1 or 2,
   wherein the exosome is an exosome obtained from stem cells or an exosome obtained from at least one selected from the group consisting of pluripotent stem cells, mesenchymal stem cells, ectoderm-derived cells, mesoderm-derived cells, and endodermderived cells.

10. A method of forming a peptide-binding hybrid liposome exosome, the method comprising:

    a step of binding a peptide to a hydrophilic part of lipid derivatives to form peptide-binding lipid derivatives;
    a step of mixing a phospholipid composition containing a phospholipid and the peptide-binding lipid derivatives to form a liposome into which the peptide-binding lipid derivatives are incorporated; and
    a step of fusing an exosome with the liposome to form a peptide-binding hybrid liposome exosome.

11. A peptide-binding hybrid liposome exosome formed by the method of forming a peptide-binding hybrid liposome exosome according to claim 10.

12. A peptide-binding hybrid liposome exosome-containing composition obtained by adding

    first primary antibodies that specifically bind to an exosome marker protein selected from CD63, CD81, and CD9,
    second primary antibodies that specifically bind to any of peptides having amino acid sequences shown in SEQ ID NOs: 1 to 5,
    first secondary antibodies conjugated with a first fluorescent dye, which specifically bind to the first primary antibodies, and
    second secondary antibodies conjugated with a second fluorescent dye, which specifically bind to the second primary antibodies,
    where the ratio of [the product of the amount of the first primary antibodies added and the antibody titer] and [the product of the amount of the second secondary antibodies added and the antibody titer] is 1:1,
    the amount ratio of the first primary antibodies and the first secondary antibodies added is 2:1, and

the amount ratio of the second primary antibodies and the second secondary antibodies added is 2:1, and when a peptide-binding hybrid liposome exosome that binds to a magnetic bead that specifically binds to a complex lipid contained in an exosome is analyzed using flow cytometry, the following condition is satisfied:

$$0.6 \leq A/(A+B+C) \leq 1.0 \quad \text{Formula (1)}$$

(where, in Formula (1), A is an area of a peak indicating the peptide-binding hybrid liposome exosome labeled with both the first fluorescent dye and the second fluorescent dye, B is an area of a peak indicating the exosome labeled with only the first fluorescent dye, and C is an area of a peak indicating the liposome labeled with only the second fluorescent dye).

13. A method of selecting a peptide-binding hybrid liposome exosome-containing composition, the method comprising:

adding
first primary antibodies that specifically bind to an exosome marker protein selected from CD63, CD81, and CD9, second primary antibodies that specifically bind to any of peptides having amino acid sequences shown in SEQ ID NOs: 1 to 5,
first secondary antibodies conjugated with a first fluorescent dye, which specifically bind to the first primary antibodies, and
second secondary antibodies conjugated with a second fluorescent dye, which specifically bind to the second primary antibodies,
where the ratio of [the product of the amount of the first primary antibodies added and the antibody titer] and [the product of the amount of the second secondary antibodies added and the antibody titer] is 1:1, the amount ratio of the first primary antibodies and the first secondary antibodies added is 2:1, and the amount ratio of the second primary antibodies and the second secondary antibodies added is 2:1; and selecting a peptide-binding hybrid liposome exosome-containing composition that satisfies the following condition when a peptide-binding hybrid liposome exosome that binds to a magnetic bead that specifically binds to a complex lipid contained in an exosome is analyzed using flow cytometry:

$$0.6A \leq /(A+B+C) \leq 1.0 \quad \text{Formula (1)}$$

(where, in Formula (1), A is an area of a peak indicating the peptide-binding hybrid liposome exosome labeled with both the first fluorescent dye and the second fluorescent dye, B is an area of a peak indicating the exosome labeled with only the first fluorescent dye, and C is an area of a peak indicating the liposome labeled with only the second fluorescent dye).

14. A peptide-binding exosome, comprising in a lipid bilayer:

an exosome comprising an exosome marker protein selected from CD63, CD81, and CD9, and peptide-binding lipid derivatives in which a peptide binds to a hydrophilic part of a complex lipid.

15. The peptide-binding exosome according to claim 14,

wherein the peptide has 10 to 30 amino acid residues,
wherein the peptide comprises a repeating sequence composed of 3 to 4 amino acid residues comprising at least one hydrophobic amino acid residue selected from an alanine residue, a leucine residue, and a methionine residue and at least one amino acid residue selected from a glutamic acid residue, an aspartic acid residue, a glutamine residue, and an asparagine residue, and
wherein at least one of amino acid residues constituting the repeating sequence is one of a glutamic acid residue, an aspartic acid residue, a glutamine residue, and an asparagine residue.

16. The peptide-binding exosome according to claim 14,

wherein the peptide has 10 to 30 amino acid residues, and
wherein the peptide has at least one basic amino acid residue.

**17.** The peptide-binding exosome according to claim 16,
wherein the peptide has 2 to 5 consecutive basic amino acid residues.

**18.** The peptide-binding exosome according to claim 16 or 17,
wherein the peptide does not contain an acidic amino acid residue.

**19.** The peptide-binding exosome according to claim 14 or 15,
wherein the peptide is any of peptides having amino acid sequences shown in SEQ ID NOs: 1 to 5.

**20.** The peptide-binding exosome according to claim 14 or 15,

wherein the complex lipid is a complex lipid having, at an end, any reactive group selected from the group consisting of primary amino groups, carboxyl groups, hydroxy groups, phosphate groups, and thiol groups, and wherein the complex lipid is at least one selected from the group consisting of sphingophospholipids, glycosphingolipids, glycerophospholipids, glyceroglycolipids, ether type phospholipids, and ether type glycolipids.

(a)

Beads - Exosome NoFusion No-pep

(A)

Exo: 64.042%

PECy7 (CD63) - PE-Cy7-H

Fig. 10 (1 of 12)

Fig. 10 (2 of 12)

Fig. 10 (3 of 12)

Fig. 10 (4 of 12)

(b)

Beads - Exosome NoFusion pep-insertion

Fig. 10 (5 of 12)

Beads - Exosome NoFusion No-pep

Fig. 10 (6 of 12)

Beads – Exosome Fusion pep-insertion

Fig. 10 (7 of 12)

Beads - Exosome Fusion pep-insertion

Fig. 10 (8 of 12)

(c)
All Events - Exosome NoFusion No-pep

Fig. 10 (9 of 12)

All Events - Exosome NoFusion pep-insertion

R1: 0.696%

Fig. 10 (10 of 12)

All Events - Exosome Fusion no-pep-insertion

Fig. 10 (11 of 12)

Fig. 10 (12 of 12)

(a)

Beads - Exosome NoFusion No-pep

(A)

Fig. 11 (1 of 12)

Fig. 11 (2 of 12)

Fig. 11 (3 of 12)

Fig. 11 (4 of 12)

(b)

Fig. 11 (5 of 12)

Fig. 11 (6 of 12)

Fig. 11 (7 of 12)

## Beads - Exosome Fusion pep-insertion

Fig. 11 (8 of 12)

(c)

All Events - Exosome NoFusion No-pep

Fig. 11 (9 of 12)

All Events - Exosome NoFusion pep-insertion

R1: 2.747%

PECy7 (CD63) - PE-Cy7-H

FITC (pep or CD63) - FITC-H

Fig. 11 (10 of 12)

All Events - Exosome Fusion no-pep-insertion

R1: 3.499%

PECy7 (CD63) - PE-Cy7-H

FITC (pep or CD63) - FITC-H

Fig. 11 (11 of 12)

All Events - Exosome Fusion pep-insertion

Fig. 11 (12 of 12)

(a)

Beads - Exosome NoFusion No-pep

Fig. 12 (1 of 12)

Beads - Exosome NoFusion pep-insertion

Fig. 12 (2 of 12)

Fig. 12 (3 of 12)

Fig. 12 (4 of 12)

(b)

**Beads - Exosome NoFusion No-pep**

Fig. 12 (5 of 12)

Fig. 12 (6 of 12)

Fig. 12 (7 of 12)

Beads - Exosome Fusion pep-insertion

Pep: 41.732%

Count

FITC (pep or CD63) - FITC-H

Fig. 12 (8 of 12)

(c)

Fig. 12 (9 of 12)

Fig. 12 (10 of 12)

All Events - Exosome Fusion no-pep-insertion

Fig. 12 (11 of 12)

All Events - Exosome Fusion pep-insertion

Fig. 12 (12 of 12)

(c)

All Events - Exosome NoFusion No-pep

(A)

R1: 30.982%

PECy7 (CD63) - PE-Cy7-H

FITC (pep or CD63) - FITC-H

Fig. 15 (1 of 4)

All Events - Exosome NoFusion pep-insertion

(B)

R1: 33.179%

y-axis: PECy7 (CD63) - PE-Cy7-H

x-axis: FITC (pep or CD63) - FITC-H

Fig. 15 (2 of 4)

(C)

All Events - Exosome Fusion no-pep-insertion

Fig. 15 (3 of 4)

Fig. 15 (4 of 4)

( c )

Fig. 16 (1 of 4)

(B)

All Events - Exosome NoFusion pep-insertion

R1: 42.890%

PECy7 (CD63) - PE-Cy7-H

FITC (pep or CD63) - FITC-H

Fig. 16 (2 of 4)

(C)

All Events - Exosome Fusion no-pep-insertion

R1: 58.255%

PECy7 (CD63) - PE-Cy7-H

FITC (pep or CD63) - FITC-H

Fig. 16 (3 of 4)

Fig. 16 (4 of 4)

## INTERNATIONAL SEARCH REPORT

| International application No. |
|---|
| **PCT/JP2022/031768** |

### A. CLASSIFICATION OF SUBJECT MATTER

*C07K 17/02*(2006.01)i; *A61K 35/12*(2015.01)i; *A61K 45/00*(2006.01)i; *A61K 47/69*(2017.01)i; *A61P 43/00*(2006.01)i; *C07K 7/06*(2006.01)i; *C07K 7/08*(2006.01)i; *C07K 14/705*(2006.01)i; *C12N 5/071*(2010.01)i; *C12N 5/0735*(2010.01)i; *C12N 5/0775*(2010.01)i; *C12N 15/11*(2006.01)i; *C12Q 1/24*(2006.01)i; *G01N 33/53*(2006.01)i
FI:  C07K17/02; A61P43/00 111; A61K45/00; A61K47/69; C07K14/705; C07K7/08; C07K7/06; C12N5/0735; C12N5/071; C12Q1/24; C12N5/0775; G01N33/53 S; C12N15/11 Z; A61K35/12 ZNA

According to International Patent Classification (IPC) or to both national classification and IPC

### B. FIELDS SEARCHED

Minimum documentation searched (classification system followed by classification symbols)

C07K17/02; A61K35/12; A61K45/00; A61K47/69; A61P43/00; C07K7/06; C07K7/08; C07K14/705; C12N5/071; C12N5/0735; C12N5/0775; C12N15/11; C12Q1/24; G01N33/53

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN); UniProt/GeneSeq; SwissProt/GeneSeq

### C. DOCUMENTS CONSIDERED TO BE RELEVANT

| Category* | Citation of document, with indication, where appropriate, of the relevant passages | Relevant to claim No. |
|---|---|---|
| X | JP 2019-218349 A (ANJARIUM BIOSCIENCES AG) 26 December 2019 (2019-12-26) claims, paragraphs [0060], [0068], [0071], examples 2, 3 | 1-12, 14-20 |
| Y | claims, paragraphs [0060], [0068], [0071], examples 2, 3 | 1-20 |
| Y | JP 2020-531018 A (CODIAK BIOSCIENCES, INC.) 05 November 2020 (2020-11-05) paragraphs [0068]-[0077] | 13 |
| A | paragraph [0068] | 1-20 |
| Y | WO 2020/219563 A1 (TCR2 THERAPEUTICS INC.) 29 October 2020 (2020-10-29) claims | 1-20 |
| Y | WO 2016/167367 A1 (KYOTO UNIV.) 20 October 2016 (2016-10-20) paragraph [0022] | 1-20 |

✓ Further documents are listed in the continuation of Box C.      ✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 October 2022** | **18 October 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
| --- |
| **PCT/JP2022/031768** |

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2017-535599 A (WISCONSIN ALUMNI RESEARCH FOUND.) 30 November 2017 (2017-11-30) <br> claims, examples 8-10 | 14-20 |
| Y | JP 2021-506795 A (BOARD OF REGENTS, THE UNIV. OF TEXAS SYSTEM) 22 February 2021 (2021-02-22) <br> claims, paragraph [0063] | 14-20 |
| Y | SEVERIC, Maja et al. Genetically-engineered anti-PSMA exosome mimetics targeting advanced prostate cancer in vitro and in vivo. Journal of Controlled Release. 14 December 2020, vol. 330, pp. 101-110 <br> abstract | 14-20 |
| A | JP 2021-503300 A (CODIAK BIOSCIENCES, INC.) 12 February 2021 (2021-02-12) <br> entire text | 1-20 |
| P, A | WO 2021/211460 A1 (CITY OF HOPE) 21 October 2021 (2021-10-21) <br> entire text | 1-20 |
| A | SATO, Yuko T. et al. Engineering hybrid exosomes by membrane fusion with liposomes. Scientific Reports. 25 February 2016, vol. 6, article no. 21933, pp. 1-11 <br> entire text | 1-20 |
| A | LEE, Junsung et al. Cellular Engineering with Membrane Fusogenic Liposomes to Produce Functionalized extracellular vesicles. Applied Materials & Interfaces. 08 March 2016, vol. 8, pp. 6790-6795 <br> entire text | 1-20 |

Form PCT/ISA/210 (second sheet) (January 2015)

**INTERNATIONAL SEARCH REPORT**

International application No.

**PCT/JP2022/031768**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

         ☑ in the form of an Annex C/ST.25 text file.

         ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

         ☐ in the form of an Annex C/ST.25 text file (Rule 13*ter*.1(a)).

         ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    The "form of an Annex CST.25 text file" is replaced with the "form of ST.26.".

Form PCT/ISA/210 (continuation of first sheet) (January 2015)

**INTERNATIONAL SEARCH REPORT**
Information on patent family members

International application No.

**PCT/JP2022/031768**

| Patent document cited in search report | | | Publication date (day/month/year) | Patent family member(s) | | Publication date (day/month/year) |
|---|---|---|---|---|---|---|
| JP | 2019-218349 | A | 26 December 2019 | US 2016/0354313 A1 claims, paragraphs [0089], [0097], [0110], examples 2, 3 WO 2015/110957 A2 EP 3096741 A2 CN 105934240 A KR 10-2016-0110410 A | | |
| JP | 2020-531018 | A | 05 November 2020 | US 2019/0060483 A1 paragraphs [0113]-[0117] WO 2019/040920 A1 EP 3672573 A1 CN 111212632 A KR 10-2020-0071065 A | | |
| WO | 2020/219563 | A1 | 29 October 2020 | EP 3959322 A1 CN 114258430 A | | |
| WO | 2016/167367 | A1 | 20 October 2016 | (Family: none) | | |
| JP | 2017-535599 | A | 30 November 2017 | US 2016/0129098 A1 claims, examples 8-10 WO 2016/073864 A1 EP 3215185 A1 | | |
| JP | 2021-506795 | A | 22 February 2021 | US 2020/0345648 A1 claims, paragraph [0080] WO 2019/118826 A1 EP 3723733 A1 CN 111479557 A KR 10-2020-0098639 A | | |
| JP | 2021-503300 | A | 12 February 2021 | US 2019/0151456 A1 whole document WO 2019/099942 A1 EP 3672614 A1 KR 10-2020-0091390 A CN 111511384 A | | |
| WO | 2021/211460 | A1 | 21 October 2021 | (Family: none) | | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021135828 A

- JP 6137894 B

**Non-patent literature cited in the description**

- *Int. J. Mol. Sci.,* 2019, vol. 20, 2167
- *Nat. Rev. Mol. Cell Biol.,* 2018, vol. 19, 281
- **NAKAI, W. et al.** A novel affinity-based method for the isolation of highly purified extracellular vesicles. *Sci. Rep.,* vol. 6, 33935

- *Journal of Japanese Biochemical Society,* 2019, vol. 91 (4), 514-518